(19) Europäisches Patentamt
European Patent Office
Office européen des brevets

(11) Veröffentlichungsnummer: **0 166 171 B1**

# (12) EUROPÄISCHE PATENTSCHRIFT

(45) Veröffentlichungstag der Patentschrift: **07.12.88**

(51) Int. Cl.⁴: **C 07 D 231/46**, C 07 D 405/12, C 07 D 405/06, A 01 N 43/56

(21) Anmeldenummer: **85106049.1**

(22) Anmeldetag: **17.05.85**

(54) **Substituierte Pyrazolin-5-one.**

(30) Priorität: **23.05.84 DE 3419136**
**21.12.84 DE 3446876**

(43) Veröffentlichungstag der Anmeldung:
**02.01.86 Patentblatt 86/1**

(45) Bekanntmachung des Hinweises auf die Patenterteilung:
**07.12.88 Patentblatt 88/49**

(84) Benannte Vertragsstaaten:
**AT BE CH DE FR GB IT LI NL SE**

(56) Entgegenhaltungen:
**US-A- 2 510 696**

**CHEMICAL ABSTRACTS, Band 97, Nr. 5, 2. August 1982, Seite 569, Nr. 38878h, Columbus, Ohio, US; T. UEDA u.a.: "Synthesis of pyrazolone derivatives. XXXX. Synthesis and analgesic activity of 4-alkoxyimino (or alkylaminomethylene)-3-methylcarbamoyl-1-methyl(or phenyl)-2-pyrazolin-5-ones", & YAKUGAKU ZASSHI 1982, 102(3), 295-9**
**CHEMICAL ABSTRACTS, Band 86, Nr. 19, 9. Mai 1977, Seiten 552,554, Nr. 139927b, Columbus, Ohio, US; W. HAENSEL u.a.: "E/Z-isomerism of substituted 4-oximinopyrazolin-5-ones", & ARCH. PHARM.**

(73) Patentinhaber: **BAYER AG, Konzernverwaltung RP Patentabteilung, D-5090 Leverkusen 1 Bayerwerk (DE)**

(72) Erfinder: **Jelich, Klaus, Dr., Pahlkestrasse 5, D-5600 Wuppertal 1 (DE)**
Erfinder: **Krämer, Wolfgang, Dr., Am Eckbusch 39/45, D-5600 Wuppertal 1 (DE)**
Erfinder: **Brandes, Wilhelm, Dr., Eichendorffstrasse 3, D-5653 Leichlingen 3 (DE)**
Erfinder: **Hänssler, Gerd, Dr., Heymannstrasse 40, D-5090 Leverkusen 1 (DE)**
Erfinder: **Reinecke, Paul, Dr., Steinstrasse 8, D-5090 Leverkusen 3 (DE)**

(56) Entgegenhaltungen: (Fortsetzung)
**(WEINHEIM, GER.) 1976, 309(11), 900-8**
**J pr (2) 50 513 (Curtins) Organic Chemistry, Bd. 50, pp. 536-538**

**Die Akte enthält technische Angaben, die nach dem Eingang der Anmeldung eingereicht wurden und die nicht in dieser Patentschrift enthalten sind.**

**EP 0 166 171 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft neue substituierte Pyrazolin-5-one, mehrere Verfahren zu ihrer Herstellung und ihre Verwendung als Schädlingsbekämpfungsmittel.

Es ist bereits bekannt, dass organische Stickstoff-Verbindungen, wie beispielsweise das Zinkethylen-1,2-bis-(dithiocarbamat), fungizide Eigenschaften besitzen (vergl. z.B. R. Wegler «Chemie der Pflanzenschutz- und Schädlingsbekämpfungsmittel», Springer Verlag Berlin, Heidelberg, New York 1970, Band 2, S. 65 f).

Weiterhin sind das 3-Methyl-4-ethoximino-5-pyrazolon bekannt (vgl. J. pr. Chemie [2], Bd. 50, Seite 513) und das 1-Phenyl-3-methyl-4-benzyloximino-5-pyrazolon (vgl. Organic Chemistry, Bd. 3, Seite 537) bekannt. Eine biologische Wirkung ist nicht bekannt.

Dass 4-Oximinopyrazol-5-one fungizide Eigenschaften besitzen, ist bereits aus US-A-2 510 696 bekannt.

Die Wirkung dieser Verbindungen ist jedoch insbesondere bei niedrigen Aufwandmengen und -konzentrationen nicht immer in allen Anwendungsbereichen völlig zufriedenstellend.

Es wurden neue substituierte Pyrazolin-5-one der allgemeinen Formel (I)

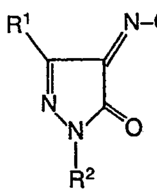

(I)

in welcher

$R^1$ für Wasserstoff oder für geradkettiges oder verzweigtes Alkyl mit 1 bis 8 Kohlenstoffatomen steht,

$R^2$ für Wasserstoff, für jeweils geradkettiges oder verzweigtes Alkyl, Alkenyl, Alkinyl, Cyanalkyl, Hydroxyalkyl, Alkoxyalkyl, Hydroxycarbonylalkyl, Alkoxycarbonylalkyl, Aminocarbonylalkyl, Alkylaminocarbonylalkyl oder Dialkylaminocarbonylalkyl mit jeweils bis zu 8 Kohlenstoffatomen in den einzelnen Alkyl- bzw. Alkenyl- oder Alkinylteilen, für Oxiranylalkyl mit 1 bis 4 Kohlenstoffatomen in geradkettigem oder verzweigtem Alkylteil, für geradkettiges oder verzweigtes, gegebenenfalls einfach oder mehrfach, gleich oder verschieden substituiertes Aralkyl mit 1 bis 4 Kohlenstoffatomen im Alkylteil und 6 bis 10 Kohlenstoffatomen im Arylteil oder für gegebenenfalls einfach oder mehrfach, gleich oder verschieden substituiertes Aryl mit 6 bis 10 Kohlenstoffatomen steht, wobei als Substituenten jeweils in Frage kommen: Halogen, Cyano, Nitro, Hydroxy, jeweils geradkettiges oder verzweigtes Alkyl, Alkoxy, Dioxyalkylen, Alkylcarbonyloxy oder Alkylthio mit jeweils bis zu 4 Kohlenstoffatomen, geradkettiges oder verzweigtes Halogenalkyl, Halogenalkoxy oder Halogenalkylthio mit jeweils bis zu 4 Kohlenstoffatomen und bis zu 9 gleichen oder verschiedenen Halogenatomen oder Phenyl und

$R^3$ für geradkettiges oder verzweigtes Alkyl mit 1 bis 20 Kohlenstoffatomen, für jeweils geradkettiges oder verzweigtes Alkenyl, Alkinyl, Cyanalkyl, Hydroxyalkyl, Alkoxyalkyl, Hydroxycarbonylalkyl oder Alkoxycarbonylalkyl mit jeweils bis zu 8 Kohlenstoffatomen in den einzelnen Alkyl- bzw. Alkenyl- oder Alkinylteilen, für Cycloalkyl mit 3 bis 7 Kohlenstoffatomen, Cycloalkylalkyl mit 3 bis 6 Kohlenstoffatomen im Cycloalkylteil und 1 oder 2 Kohlenstoffatomen im Alkylteil oder für jeweils gegebenenfalls einfach oder mehrfach, gleich oder verschieden substituiertes Arylalkyl, Aryloxyalkyl oder Arylthioalkyl mit 1 bis 4 Kohlenstoffatomen in den einzelnen Alkylteilen und 6 bis 10 Kohlenstoffatomen in den Arylteilen steht, wobei als Substituenten die bei $R^2$ genannten in Frage kommen, wobei jedoch $R^3$ nur dann für Methyl oder Ethyl steht, wenn $R^1$ und/oder $R^2$ nicht gleichzeitig für Wasserstoff oder Methyl stehen, weiterhin ist ausgenommen die Verbindung 1-Phenyl-3-methyl-4-benzyloximino-5-pyrazolon,
gefunden.

Die Verbindungen der Formel (I) können als geometrische Isomere oder Isomerengemische unterschiedlicher Zusammensetzung vorliegen. Sowohl die reinen Isomeren als auch die Isomerengemische werden erfindungsgemäss beansprucht.

Weiterhin wurde gefunden, dass man die neuen substituierten Pyrazolin-5-one der allgemeinen Formel (I)

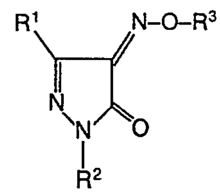

(I)

in welcher

$R^1$ für Wasserstoff oder für geradkettiges oder verzweigtes Alkyl mit 1 bis 8 Kohlenstoffatomen steht,

$R^2$ für Wasserstoff, für jeweils geradkettiges oder verzweigtes Alkyl, Alkenyl, Alkinyl, Cyanalkyl, Hydroxyalkyl, Alkoxyalkyl, Hydroxycarbonylalkyl, Alkoxycarbonylalkyl, Aminocarbonylalkyl, Alkylaminocarbonylalkyl oder Dialkylaminocarbonylalkyl mit jeweils bis zu 8 Kohlenstoffatomen in den einzelnen Alkyl- bzw. Alkenyl- oder Alkinylteilen, für Oxiranylalkyl mit 1 bis 4 Kohlenstoffatomen in geradkettigem oder verzweigtem Alkylteil, für geradkettiges oder verzweigtes, gegebenenfalls einfach oder mehrfach, gleich oder verschieden substituiertes Aralkyl mit 1 bis 4 Kohlenstoffatomen im Alkylteil und 6 bis 10 Kohlenstoffatomen im Arylteil oder für gegebenenfalls einfach oder mehrfach, gleich oder verschieden substituiertes Aryl mit 6 bis 10 Kohlenstoffatomen steht, wobei als Substituenten jeweils in Frage kommen: Halogen, Cyano, Nitro, Hydroxy, jeweils geradkettiges oder verzweigtes Alkyl, Alkoxy, Dioxyalkylen, Alkylcarbonyloxy oder Alkylthio mit jeweils bis zu 4 Kohlenstoffatomen, geradkettiges oder verzweigtes Halogenalkyl, Halogenalkoxy oder

Halogenalkylthio mit jeweils bis zu 4 Kohlenstoffatomen und bis zu 9 gleichen oder verschiedenen Halogenatomen oder Phenyl und

$R^3$ für geradkettiges oder verzweigtes Alkyl mit 1 bis 20 Kohlenstoffatomen, für jeweils geradkettiges oder verzweigtes Alkenyl, Alkinyl, Cyanalkyl, Hydroxyalkyl, Alkoxyalkyl, Hydroxycarbonylalkyl oder Alkoxycarbonylalkyl mit jeweils bis zu 8 Kohlenstoffatomen in den einzelnen Alkyl- bzw. Alkenyl- oder Alkinylteilen, für Cycloalkyl mit 3 bis 7 Kohlenstoffatomen, Cycloalkylalkyl mit 3 bis 6 Kohlenstoffatomen im Cycloalkylteil und 1 oder 2 Kohlenstoffatomen im Alkylteil oder für jeweils gegebenenfalls einfach oder mehrfach, gleich oder verschieden substituiertes Arylalkyl, Aryloxyalkyl oder Arylthioalkyl mit 1 bis 4 Kohlenstoffatomen in den einzelnen Alkylteilen und 6 bis 10 Kohlenstoffatomen in den Arylteilen steht, wobei als Substituenten die bei $R^2$ genannten in Frage kommen, wobei jedoch $R^3$ nur dann für Methyl oder Ethyl steht, wenn $R^1$ und/oder $R^2$ nicht gleichzeitig für Wasserstoff oder Methyl stehen, weiterhin ist ausgenommen die Verbindung 1-Phenyl-3-methyl-4-benzyloximino-5-pyrazolon,

erhält, wenn man

(a) 4-Oximino-pyrazolin-5-one der Formel (II)

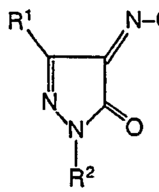

(II)

in welcher

$R^1$ und $R^2$ die oben angegebene Bedeutung haben und

A für Wasserstoff oder ein Alkalimetallkation steht,

mit Alkylierungsmitteln der Formel (III)

$$R^3\text{–}X \qquad (III)$$

in welcher

$R^3$ die oben angegebene Bedeutung hat und

X für eine elektronenanziehende Abgangsgruppe steht,

gegebenenfalls in Gegenwart eines Verdünnungsmittels, gegebenenfalls in Gegenwart eines Säurebindemittels und gegebenenfalls in Gegenwart eines Phasentransfer-Katalysators umsetzt, oder wenn man

(b) die nach Verfahren (a) erhältlichen 4-Alkoximino-pyrazolin-5-one der Formel (Ia)

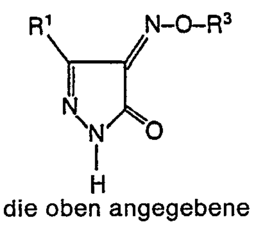

(Ia)

in welcher

$R^1$ und $R^3$ die oben angegebene Bedeutung haben,

mit Alkylierungsmitteln der Formel (IV)

$$R^{2'}\text{–}Y \qquad (IV)$$

in welcher

$R^{2'}$ für Alkyl, Alkenyl, Alkinyl, Cyanalkyl, Hydroxyalkyl, Alkoxyalkyl, Hydroxycarbonylalkyl, Alkoxycarbonylalkyl oder für jeweils gegebenenfalls substituiertes Aralkyl oder Aryl steht und

Y für eine elektronenanziehende Abgangsgruppe steht,

gegebenenfalls in Gegenwart eines Verdünnungsmittels, gegebenenfalls in Gegenwart eines Säurebindemittels und gegebenenfalls in Gegenwart eines Phasentransfer-Katalysators umsetzt.

Schliesslich wurde gefunden, dass die neuen substituierten Pyrazolin-5-one der Formel (I) fungizide und bakterizide Eigenschaften besitzen.

Überraschenderweise zeigen die neuen substituierten Pyrazolin-5-one der Formel (I) bessere fungizide Eigenschaften als das aus dem Stand der Technik bekannte Zinkethylen-1,2-bis-dithiocarbamat, welches wirkungsmässig eine naheliegende Verbindung ist.

Die erfindungsgemässen Pyrazolin-5-one sind durch die Formel (I) allgemein definiert. Bevorzugt sind Verbindungen der Formel (I), bei welchen

$R^1$ für Wasserstoff, Methyl, Ethyl, n- oder i-Propyl und n-, i-, s- oder t-Butyl steht,

$R^2$ für Wasserstoff, Methyl, Ethyl, n- oder i-Propyl, n-, i-, s- oder t-Butyl, Allyl, Butenyl, Propargyl, Cyanmethyl, Cyanethyl, Hydroxymethyl, Hydroxyethyl, Methoxymethyl, Methoxyethyl, Ethoxymethyl, Ethoxyethyl, Hydroxycarbonylmethyl, Hydroxycarbonylethyl, Methoxycarbonylmethyl, Methoxycarbonylethyl, Ethoxycarbonylmethyl, Ethoxycarbonylethyl, Aminocarbonylmethyl, Methylaminocarbonylmethyl, Ethylaminocarbonylethyl, Ethylaminocarbonylmethyl, Dimethylaminocarbonylmethyl, Diethylaminocarbonylmethyl, Aminocarbonylethyl, Oxiranylmethyl, Oxiranylethyl oder für jeweils gegebenenfalls ein- bis dreifach, gleich oder verschieden substituiertes Phenyl oder Benzyl steht, wobei als Substituenten in Frage kommen: Fluor, Chlor, Brom, Iod, Cyano, Nitro, Hydroxy, Methyl, Ethyl, n- oder i-Propyl, n-, i-, s- oder t-Butyl, Methoxy, Ethoxy, Dioxymethylen, Dioxethylen, Methylthio, Ethylthio, Acetoxy oder Propionyloxy, Chlormethyl, Dichlormethyl, Trichlormethyl, Trifluormethyl, Dichlorfluormethyl, Chlordifluormethyl, Trifluormethoxy, Trifluormethylthio oder Phenyl und

$R^3$ für Methyl, Ethyl, n- oder i-Propyl, n- oder i-Butyl, n- oder i-Pentyl, n- oder i-Hexyl, n- oder i-Heptyl, n- oder i-Octyl, n- oder i-Nonyl, n- oder i-Decyl, n- oder i-Dodecyl, n- oder i-Hexadecyl, Allyl, Butenyl, Propargyl, Cyanmethyl, Cyanethyl, Hydroxymethyl, Hydroxyethyl, Methoxymethyl, Methoxyethyl, Ethoxymethyl, Ethoxyethyl, Hydroxycarbonylmethyl, Hydroxycarbonylethyl, Methoxycarbonylmethyl, Methoxycarbonylethyl, Ethoxycarbonylmethyl, Ethoxycarbonylethyl, Cyclopropyl, Cyclopentyl, Cyclohexyl, Cyclohexylmethyl oder für einen gegebenenfalls ein- bis dreifach, gleich oder verschieden substituierten Rest der Formel

$Ar-(Z)_m-CH_2)_n-$, $Ar-(Z)_m-CH-$
$CH_3$

oder

$Ar-(Z)_m-CH-$ steht, wobei
$CN$

Ar jeweils für Phenyl oder Naphthyl steht,
Z jeweils für Sauerstoff oder Schwefel steht,
m jeweils für 0 oder 1 steht und
n für 1, 2 oder 3 steht,

und wobei als Substituenten die bei $R^2$ genannten in Frage kommen, mit der Einschränkung, dass $R^3$ nur dann für Methyl oder Ethyl steht, wenn $R^1$ und $R^2$ nicht gleichzeitig für Wasserstoff oder Methyl stehen, weiterhin ist ausgenommen die Verbindung 1-Phenyl-3-methyl-4-benzyloximino-5-pyrazolon.

Im einzelnen seien die folgenden Verbindungen der allgemeinen Formel (I) genannt:

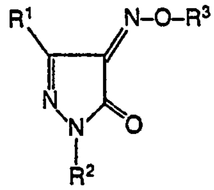

(I)

| $R^1$ | $R^2$ | $R^3$ |
|---|---|---|
| $-CH_3$ | $-CH_3$ | 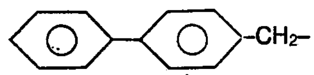 |
| $-CH_3$ | $-CH_3$ | 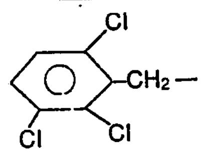 |
| $-C_2H_5$ | $-CH_3$ | 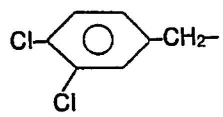 |
| $-C_2H_5$ | $-CH_3$ | 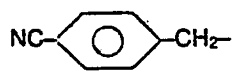 |
| $-C_2H_5$ | $-CH_3$ | 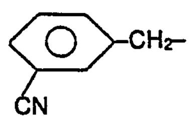 |
| $-C_3H_7$-n | $-CH_3$ | 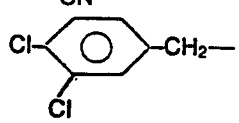 |
| $-C_3H_7$-n | $-CH_3$ | 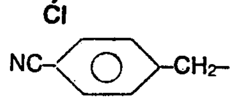 |
| $-C_3H_7$-n | $-CH_3$ | 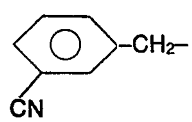 |
| $-C_4H_9$-n | $-CH_3$ | 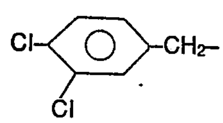 |
| $-C_4H_9$-n | $-CH_3$ | 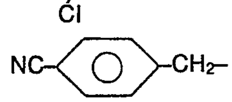 |
| $-C_4H_9$-n | $-CH_3$ | 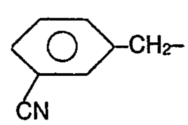 |

| $R^1$ | $R^2$ | $R^3$ |
|---|---|---|
| $-C_2H_5$ | $-CH_2-CN$ | 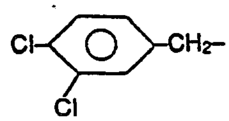 |
| $-C_3H_7-n$ | $-CH_2-CN$ | 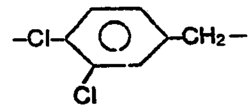 |
| $-C_4H_9-n$ | $-CH_2-CN$ | 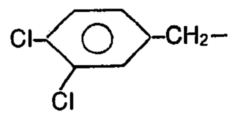 |
| $-CH_3$ | $-CH_3$ | 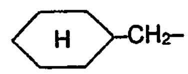 |

Verwendet man als Ausgangsstoffe beispielsweise 4-Hydroximino-1,3-dimethyl-pyrazolin-5-on und 2,6-Dichlorbenzylchlorid, so lässt sich der Reaktionsablauf des erfindungsgemässen Verfahrens (a) durch das folgende Formelschema darstellen:

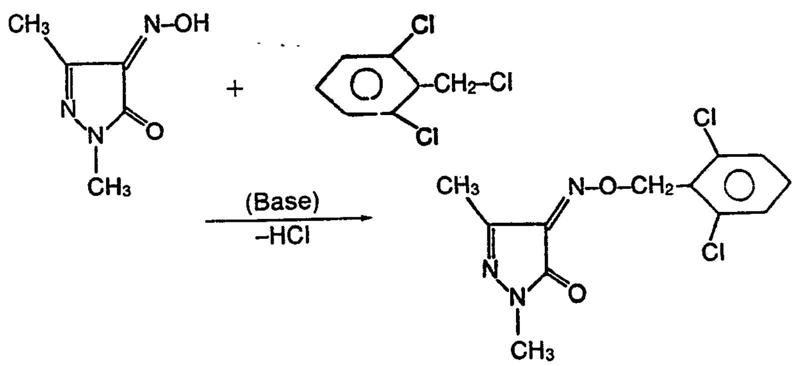


Verwendet man als Ausgangsstoffe beispielsweise 4-(2-Chlorbenzyloximino)-3-methyl-pyrazolin-5-on und Chloracetonitril, so lässt sich der Reaktionsablauf des erfindungsgemässen Verfahrens (b) durch das folgende Formelschema darstellen:

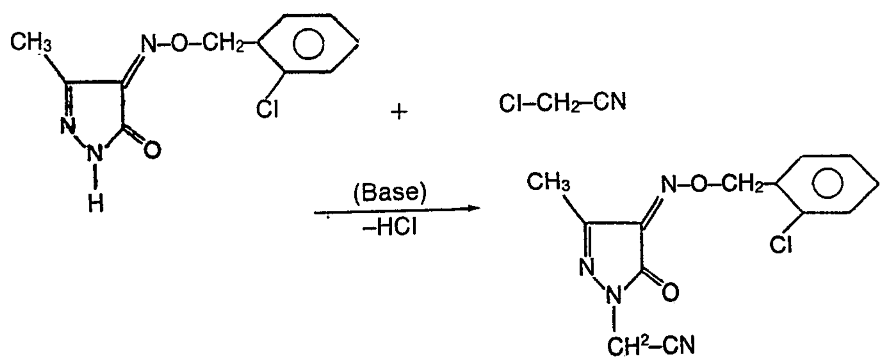


Die zur Durchführung des erfindungsgemässen Verfahrens (a) als Ausgangsstoffe benötigten 4-Oximino-pyrazolin-5-one sind durch die Formel (II) allgemein definiert. In dieser Formel (II) stehen $R^1$ und $R^2$ vorzugsweise für diejenigen Reste, die schon bei der Beschreibung der erfindungsgemässen Stoffe der Formel (I) als bevorzugt für diese Substituenten genannt wurden. A steht vorzugsweise für Wasserstoff oder für ein Natrium- oder Kaliumkation.

Die 4-Oximino-pyrazolin-5-one der Formel (II) sind teilweise bekannt [vergl. z.B. Ber. dtsch. chem. Ges. 29, 249 (1896); Coll. Czech. Chem. Commun. 25, 55 (1960); Arch. Pharm. 309, 900 (1976); Liebigs Ann. Chem. 1976, 1380]. Man erhält sie beispielsweise, wenn man β-Ketoester der Formel (V)

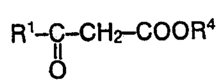 (V)

in welcher

$R^1$ die oben angegebene Bedeutung hat und

$R^4$ für niederes Alkyl, insbesondere für Methyl oder Ethyl, steht,

oder Ethoxymethylenmalonester der Formel (Va)

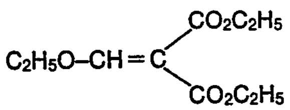

(Va)

zunächst in einer 1. Stufe mit Hydrazinen der Formel (VI)

$$R^2\text{–NH–NH}_2 \qquad \text{(VI)}$$

in welcher

$R^2$ die oben angegebene Bedeutung hat,

gegebenenfalls in Gegenwart eines Verdünnungsmittels, wie beispielsweise Ethanol, bei Temperaturen zwischen 0 °C und 100 °C cyclisiert, die aus dem Malonester der Formel (Va) sich ergebenden 4-Ethoxycarbonylpyrazolin-5-one der Formel (VIIa),

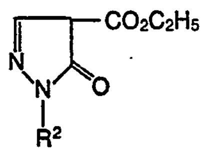

(VIIa)

in welcher

$R^2$ die oben angegebene Bedeutung hat,

in einem Zwischenschritt, nach üblichen Methoden, beispielsweise mit wässriger Salzsäure bei Temperaturen zwischen 50 °C und 120 °C verseift und decarboxyliert, und die so erhältlichen Pyrazolin-5-one der Formel (VII)

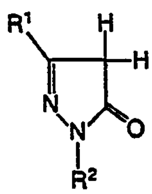

.(VII)

in welcher

$R^1$ und $R^2$ die oben angegebene Bedeutung haben,

mit einem Nitrosierungsmittel, wie beispielsweise Isopentylnitrit oder Natriumnitrit, gegebenenfalls in Gegenwart eines Verdünnungsmittels, wie beispielsweise Ethanol, Wasser oder wässrige Salzsäure, und gegebenenfalls in Gegenwart einer Base, wie beispielsweise Natriumethylat, bei Temperaturen zwischen −20 °C und +50 °C umsetzt.

Die zur Durchführung des erfindungsgemässen Verfahrens (a) weiterhin als Ausgangsstoffe benötigten Alkylierungsmittel sind durch die Formel (III) allgemein definiert. In dieser Formel (III) steht $R^3$ vorzugsweise für diejenigen Reste, die schon bei der Beschreibung der erfindungsgemässen Stoffe der Formel (I) als bevorzugt für diese Substituenten genannt wurden. X steht vorzugsweise für Halogen, insbesondere Chlor, Brom oder Iod, oder für gegebenenfalls substituiertes Alkylsulfonyloxy, Alkoxysulfonyloxy oder Arylsulfonyloxy, wie beispielsweise Methansulfonyloxy, Methoxysulfonyloxy, Trifluormethansulfonyloxy oder p-Toluolsulfonyloxy.

Die Alkylierungsmittel der Formel (III) sind allgemein bekannte Verbindungen der organischen Chemie.

Die β-Ketoester der Formel (V) bzw. der Ethoxymethylenmalonester der Formel (Va) sind ebenfalls allgemein bekannt.

Die zur Durchführung des erfindungsgemässen Verfahrens (b) als Ausgangsstoffe benötigten 4-Alkoximinopyrazolin-5-one sind durch die Formel (Ia) allgemein definiert. In dieser Formel (Ia) stehen $R^1$ und $R^3$ vorzugsweise für diejenigen Reste, die bereits bei der Beschreibung der erfindungsgemässen Stoffe der Formel (I) als bevorzugt für diese Substituenten genannt wurden.

Die 4-Alkoximinopyrazolin-5-one der Formel (Ia) sind erfindungsgemässe Verbindungen und erhältlich mit Hilfe des erfindungsgemässen Verfahrens (a).

Die zur Durchführung des erfindungsgemässen Verfahrens (b) weiterhin als Ausgangsstoffe benötigten Alkylierungsmittel sind durch die Formel (IV) allgemein definiert. In dieser Formel (IV) steht $R^{2'}$ vorzugsweise für diejenigen Reste, die bereits bei der Beschreibung der erfindungsgemässen Stoffe der Formel (I) als bevorzugt für den Substituenten $R^2$ genannt wurden, mit Ausnahme des Wasserstoffrestes. Y steht vorzugsweise für diejenigen Abgangsgruppen, die bereits bei der Beschreibung der Alkylierungsmittel der Formel (III) als bevorzugt für den Substituenten X genannt wurden.

Die Alkylierungsmittel der Formel (IV) sind ebenfalls allgemein bekannte Verbindungen der organischen Chemie.

Als Verdünnungsmittel zur Durchführung der erfindungsgemässen Verfahren (a) und (b) kommen inerte organische Lösungsmittel oder wässrige Systeme in Frage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff; Ether, wie Diethylether, Dioxan, Tetrahydrofuran oder Ethylenglykoldimethyl- oder -diethylether; Ketone, wie Aceton oder Butanon; Nitrile, wie Acetonitril oder Propionitril; Amide, wie Dimethylformamid, Dimethylacetamid, N-Methylformanilid, N-Methylpyrrolidon oder Hexamethylphosphorsäuretriamid; Ester, wie Essigsäureethylester; Sulfoxide, wie Dimethylsulfoxid, oder auch Wasser oder wässrigorganische Zweiphasen-Gemische, wie Dichlormethan-Wasser oder Toluol-Wasser.

Die erfindungsgemässen Verfahren (a) und (b) werden gegebenenfalls in Gegenwart eines Säurebindemittels durchgeführt.

Als solche kommen alle üblichen anorganischen oder organischen Basen in Frage. Hierzu gehören beispielsweise Alkalimetallhydroxide, -amide, -alkoholate oder -hydride, wie Natriumhydroxid oder Kaliumhydroxid, Natriummethylat oder Kalium-t-butylat, Natriumhydrid oder Natriumamid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemässen Verfahren (a) und (b) in einem grösseren Bereich variiert werden. Im allgemeinen arbeitet man bei Temperaturen zwischen −20 °C und +200 °C, vorzugsweise bei Temperaturen zwischen 0 °C und +150 °C.

Bei der Durchführung der erfindungsgemässen Verfahren (a) und (b) setzt man pro Mol an 4-Oximino-pyrazolin-5-on der Formeln (II) bzw. (Ia) im allgemeinen 1,0 bis 3,0 Mol, vorzugsweise 1,0 bis 1,5 Mol, an Alkylierungsmittel der Formeln (III) bzw. (IV) und gegebenenfalls 1,0 bis 3,0 Mol, vorzugsweise 1,0 bis 1,5 Mol, an Säurebindemittel ein.

Führt man die Umsetzung in einem organischwässrigen Zweiphasensystem durch, kann man gegebenenfalls in Gegenwart von 0,1 bis 1 Mol eines geeigneten Phasentransferkatalysators, wie beispielsweise einer quartären Ammonium- oder Phosphoniumverbindung arbeiten. Beispielhaft seien Triethylbenzylammoniumchlorid und Benzyl-dodecyl-dimethylammoniumchlorid genannt.

Die Aufarbeitung und Isolierung der Reaktionsprodukte der Formel (I) erfolgt nach üblichen Methoden.

Die erfindungsgemässen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:

Botrytis-Arten,
wie beispielsweise Botrytis cinerea;
Plasmopara-Arten,
wie beispielsweise Plasmopara viticola;
Uromyces-Arten,
wie beispielsweise Uromyces appendiculatus;
Sphaerotheca-Arten,
wie beispielsweise Sphaerotheca fuliginea;
Venturia-Arten,
wie beispielsweise Venturia inaequalis;
Podosphaera-Arten,
wie beispielsweise Podosphaera leucotricha;
Phytophthora-Arten,
wie beispielsweise Phytophthora infestans;
Erysiphe-Arten,
wie beispielsweise Erysiphe graminis;
Puccinia-Arten,
wie beispielsweise Puccinia recondita;
Fusarium-Arten,
wie beispielsweise Fusarium culmorum;
Ustilago-Arten,
wie beispielsweise Ustilago nuda oder Ustilago avenae;
Septoria-Arten,
wie beispielsweise Septoria nodorum;
Tilletia-Arten,
wie beispielsweise Tilletia caries;
Xanthomonas-Arten,
wie beispielsweise Xanthomonas oryzae;
Pseudomonas-Arten,
wie beispielsweise Pseudomonas lachrymans;
Pyricularia-Arten,
wie beispielsweise Pyricularia oryzae;
Pellicularia-Arten,
wie beispielsweise Pellicularia sasakii und
Pyrenophora-Arten,
wie beispielsweise Pyrenophora teres.
(Konidienform: Drechslera, Syn: Helminthosporium);
Leptosphaeria-Arten,
wie beispielsweise Leptosphaeria nodorum;
Cochliobolus-Arten,
wie beispielsweise Cochliobolus sativus
(Konidienform: Drechslera, Syn: Helminthosporium) und
Cercospora-Arten,
wie beispielsweise Cercospora canescens.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei können die erfindungsgemässen Wirkstoffe mit besonders gutem Erfolg zur Bekämpfung von Getreidekrankheiten, wie beispielsweise verursacht durch Cochliobolus sativus, Leptosphaeria nodorum, Fusarium-Arten oder Pyrenophora teres, gegen Rosterreger, gegen Mehltauerreger, zur Bekämpfung von Gemüsekrankheiten, wie beispielsweise gegen den Erreger der Tomatenbraunfäule (Phytophthora infestans) oder gegen den Erreger des Bohnengrauschimmels (Botrytis-cinerea) oder zur Bekämpfung von Reiskrankheiten, wie beispielsweise gegen den Erreger der Reisfleckenkrankheit (Pyricularia oryzae) eingesetzt werden. Dabei zeigen die erfindungsgemässen Wirkstoffe neben protektiver Wirksamkeit auch systemische Eigenschaften.

Die erfindungsgemässen Wirkstoffe zeigen neben einer hervorragenden protektiven Wirksamkeit auch sehr gute systemische Eigenschaften.

Sie zeichnen sich durch breite fungizide in-vitro-Wirkung und durch zusätzliche bakterizide Eigenschaften aus.

Die Wirkstoffe können in die üblichen Formulierungen übergeführt werden, wie Lösungen, Emulsionen, Suspensionen, Pulver, Stäubemittel, Schäume, Pasten, lösliche Pulver, Granulate, Aerosole, Suspension-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweisshydrolysate. Als Dispergiermittel kommen in Frage: z.B. Lignin-Sulfitablaugen und Methylcellulose.

Es können in den Formulierungen Haffmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Giessen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem grösseren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

Herstellungsbeispiele

Beispiel 1

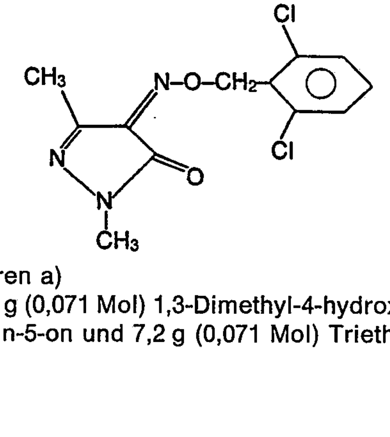

(Verfahren a)

Zu 10 g (0,071 Mol) 1,3-Dimethyl-4-hydroximino-pyrazolin-5-on und 7,2 g (0,071 Mol) Triethylamin

in 50 ml absolutem Dimethylformamid gibt man unter Rühren bei Raumtemperatur tropfenweise 13,9 g (0,071 Mol) 2,6-Dichlorbenzylchlorid in 30 ml absolutem Dimethylformamid und rührt nach beendeter Zugabe weitere 24 Stunden bei Raumtemperatur.

Zur Aufarbeitung giesst man den Ansatz in 300 ml Wasser und extrahiert viermal mit jeweils 70 ml Chloroform. Die vereinigten organischen Phasen werden fünfmal mit jeweils 200 ml Wasser gewaschen, über Magnesiumsulfat getrocknet und im Vakuum eingeengt. Der kristalline Rückstand wird mit wenig Ether verrieben und abgesaugt. Man erhält 9 g (42% der Theorie) an 1,3-Dimethyl-4-(2,6-dichlorbenzyloximino)-pyrazolin-5-on vom Schmelzpunkt 132 °C.

Herstellung der Ausgangsverbindung:

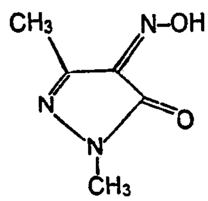

Zu 175 g (1,56 Mol) 1,3-Dimethyl-pyrazolin-5-on und 84,4 g (1,56 Mol) Natriummethylat in 1 l absolutem Ethanol gibt man unter Rühren und Eiskühlung tropfenweise 183 g (1,56 Mol) Isopentylnitrit, so dass die Innentemperatur 25 °C bis 30 °C nicht übersteigt. Nach beendeter Zugabe rührt man weitere 24 Stunden bei Raumtemperatur und saugt das ausgefallene Natriumsalz des 1,3-Dimethyl-4-hydroximino-pyrazolin-5-ons ab. Das kristalline Produkt wird in 1 l Wasser gelöst und mit Eisessig angesäuert. Zur vollständigen Fällung kühlt man für mehrere Stunden auf 0 °C und saugt dann das Produkt ab. Man erhält 162 g (74% der Theorie) an 1,3-Dimethyl-4-hydroximino-pyrazolin-5-on vom Schmelzpunkt 93 °C.

Beispiel 2

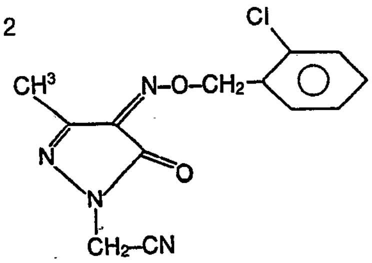

Zu 1,6 g (0,053 Mol) Natriumhydrid (80prozentige Suspension in Mineralöl) in 30 ml absolutem Dimethylformamid tropft man bei 0 °C unter Rühren und gleichzeitigem Überleiten eines trockenen Stickstoffstroms 10 g (0,04 Mol) 4-(2-Chlorbenzyloximino)-3-methylpyrazolin-5-on in 50 ml absolutem Dimethylformamid und rührt nach beendeter Zugabe eine weitere Stunde bei 0 °C. Danach tropft man ebenfalls bei 0 °C 3,6 g (0,048 Mol) Chloracetonitril zu, rührt weitere 3 Stunden bei 0 °C und anschliessend über Nacht bei Raumtemperatur.

Zur Aufarbeitung zerstört man überschüssiges Natriumhydrid durch vorsichtige Zugabe von Wasser und extrahiert anschliessend viermal mit jeweils 80 ml Chloroform. Die vereinigten organischen Phasen werden fünfmal mit jeweils 200 ml Wasser gewaschen, über Magnesiumsulfat getrocknet und im Vakuum eingeengt.

Das zurückbleibende Öl wird säulenchromatographisch (Kieselgel/Dichlormethan-Ether 1:1) gereinigt und das so erhältliche Öl durch Verreiben mit Ether kristallisiert und abgesaugt. Man erhält 3,7 g (32% der Theorie) an 4-(2-Chlorbenzyloximino)-1-cyanmethyl-3-methyl-pyrazolin-5-on vom Schmelzpunkt 118–125 °C.

In entsprechender Weise und gemäss den allgemeinen Herstellungsangaben erhält man die folgenden Verbindungen der allgemeinen Formel (I):

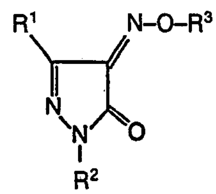

(I)

| Bsp.-Nr. | $R^1$ | $R^2$ | $R^3$ | Phys. Daten [F = Schmelzpunkt in °C] |
|---|---|---|---|---|
| 3 | $CH_3$ | $-CH_2-C\equiv CH$ | $CH_3$ | $^1$H-NMR*) 4,3 ppm |
| 4 | $(CH_3)_3C-$ | H | $CH_3$ | $^1$H-NMR*) 4,3 ppm |
| 5 | $CH_3$ | H | $-CH_2-CH=CH_2$ | F 56 |
| 6 | $CH_3$ | 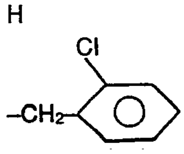 | $CH_3$ | F 96 |
| 7 | $CH_3$ | H | 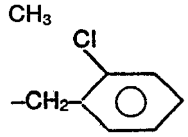 | F 130 |
| 8 | $CH_3$ | $CH_3$ | $-CH_2-CH=CH_2$ | $^1$H-NMR*) 4,9 ppm |
| 9 | $CH_3$ | $CH_3$ | 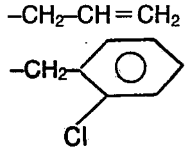 | F 104 |

| Bsp.-Nr. | $R^1$ | $R^2$ | $R^3$ | Phys. Daten [F = Schmelzpunkt in °C] |
|---|---|---|---|---|
| 10 | $CH_3$ | $-CH_2-CH=CH_2$ | $CH_3$ | $^1H$-NMR*) 4,3 ppm |
| 11 | $CH_3$ | $-CH_2-CN$ | $CH_3$ | $^1H$-NMR*) 4,3 ppm |
| 12 | $CH_3$ | H | 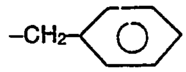 | $^1H$-NMR*) 5,5 ppm |
| 13 | $CH_3$ | H | 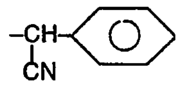 | $^1H$-NMR*) 2,1 ppm |
| 14 | $CH_3$ | $CH_3$ | 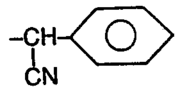 | F 167 |
| 15 | $CH_3$ | $-CH_2-OC_2H_5$ | 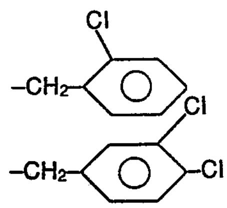 | $^1H$-NMR*) 5,0 und 5,6 ppm |
| 16 | $CH_3$ | $CH_3$ | | F 98 |
| 17 | $CH_3$ | $CH_3$ | 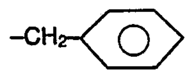 | $^1H$-NMR*) 5,5 ppm |
| 18 | $CH_3$ | $CH_3$ | 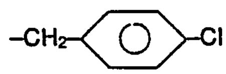 | F 98 |
| 19 | $CH_3$ | $CH_3$ | 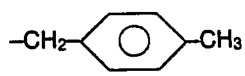 | $^1H$-NMR*) 5,4 ppm |
| 20 | $CH_3$ | $CH_3$ | 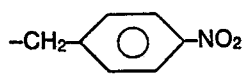 | F 130 |
| 21 | $CH_3$ | $CH_3$ | 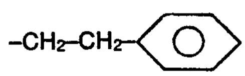 | $^1H$-NMR*) 4,7 ppm |
| 22 | $CH_3$ | $CH_3$ | $-CH_2-C\equiv CH$ | F 110–112 |
| 23 | $CH_3$ | $-CH(CH_3)_2$ | 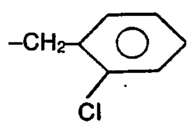 | F 95 |
| 24 | $CH_3$ | $-CH_2-COOC_2H_5$ | 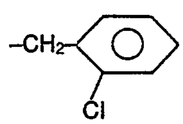 | $^1H$-NMR*) 4,5 und 5,6 ppm |
| 25 | $CH_3$ | $-CH_2-CH_2-OH$ | 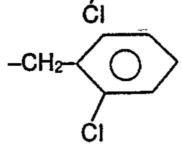 | F89 |
| 26 | $CH_3$ | H | 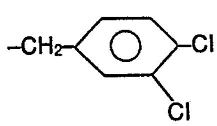 | F 147 |
| 27 | $CH_3$ | $-CH_2-COOH$ | 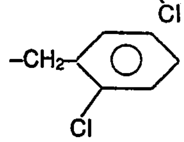 | F 118–125 |
| 28 | $CH_3$ | $CH_3$ | $-(CH_2)_4-CH_3$ | $^1H$-NMR*) 4,5 ppm |
| 29 | $CH_3$ | $CH_3$ | $-(CH_2)_2-CH_3$ | $^1H$-NMR*) 4,4 ppm |
| 30 | $CH_3$ | $CH_3$ | $-CH(CH_3)_2$ | F 86 |
| 31 | $CH_3$ | $CH_3$ | $-CH_2-CH_2-CH(CH_3)_2$ | $^1H$-NMR*) 4,5 ppm |

| Bsp.-Nr. | $R^1$ | $R^2$ | $R^3$ | Phys. Daten [F = Schmelzpunkt in °C] |
|---|---|---|---|---|
| 32 | $CH_3$ | $CH_3$ | $-CH_2-COOC_2H_5$ | F 66–68 |
| 33 | $CH_3$ | $CH_3$ | 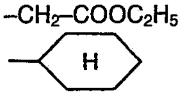 | $^1$H-NMR$^{+)}$ 4,5 ppm |
| 34 | $CH_3$ | H | 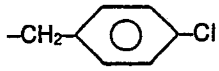 | F 146–151 |
| 35 | $CH_3$ | $CH_3$ | $-(CH_2)_3-CH_3$ | $^1$H-NMR$^{*)}$ 4,5 ppm |
| 36 | $CH_3$ | $CH_3$ | $-(CH_2)_{11}-CH_3$ | $^1$H-NMR$^{*)}$ 4,5 ppm |
| 37 | $CH_3$ | H | $-CH(CH_3)_2$ | F 92 |
| 38 | H | $CH_3$ | 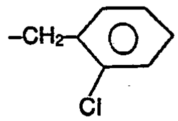 | F 77–80 |
| 39 | $CH_3$ | H | 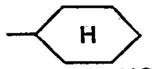 | F 100 |
| 40 | $CH_3$ | $CH_3$ | 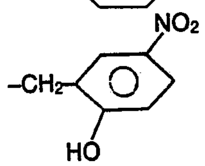 | F 137 |
| 41 | $CH_3$ | H | $-(CH_2)_3-CH_3$ | F 65–68 |
| 42 | $CH_3$ | H | $-(CH_2)_{11}-CH_3$ | F 77 |
| 43 | $CH_3$ | $CH_3$ | 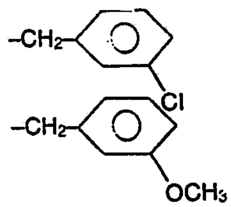 | F 90 |
| 44 | $CH_3$ | $CH_3$ | | $^1$H-NMR$^{*)}$ 5,4 ppm |
| 45 | $CH_3$ | $CH_3$ | 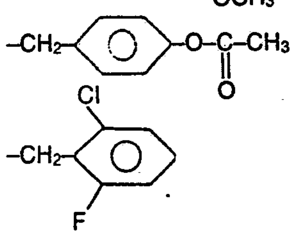 | F 101–104 |
| 46 | $CH_3$ | $CH_3$ | | F 144 |
| 47 | $CH_3$ | $CH_3$ | 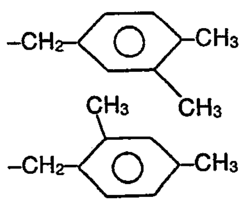 | F 84–88 |
| 48 | $CH_3$ | $CH_3$ | | $^1$H-NMR$^{*)}$ 5,4 ppm |
| 49 | $CH_3$ | $CH_3$ | 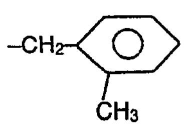 | F 102 |
| 50 | $CH_3$ | $CH_3$ | 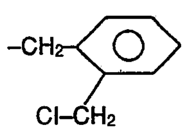 | $^1$H-NMR$^{*)}$ 5,6 ppm |
| 51 | $CH_3$ | $CH_3$ | 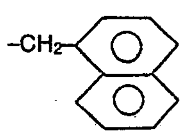 | F119 |

| Bsp.-Nr. | $R^1$ | $R^2$ | $R^3$ | Phys. Daten [F = Schmelzpunkt in °C] |
|---|---|---|---|---|
| 52 | $CH_3$ | $C_2H_3$ | 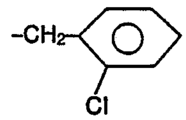 | $^1$H–NMR$^{*)}$ 5,6 ppm |
| 53 | $CH_3$ | $CH_3$ | 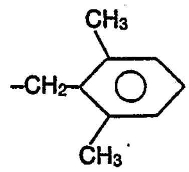 | $^1$H–NMR$^{*)}$ 5,4 ppm |
| 54 | $CH_3$ | $CH_3$ | $-(CH_2)_5-CH_3$ | $^1$H–NMR$^{*)}$ 4,5 ppm |
| 55 | $CH_3$ | $CH_3$ | $-(CH_2)_7-CH_3$ | $^1$H–NMR$^{*)}$ 4,5 ppm |
| 56 | $CH_3$ | $CH_3$ | $-(CH_2)_9-CH_3$ | $^1$H–NMR$^{*)}$ 4,5 ppm |
| 57 | $CH_3$ | $CH_3$ | $-(CH_2)_{15}-CH_3$ | F 28 |
| 58 | $CH_3$ | $CH_3$ | 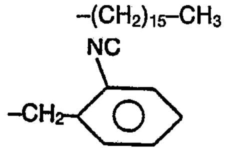 | F 126 |
| 59 | $CH_3$ | $CH_3$ | 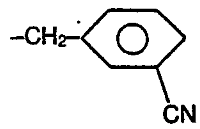 | F 126 |
| 60 | $CH_3$ | $CH_3$ | 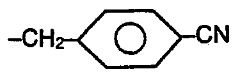 | F 158 |
| 61 | $CH^3$ | $CH_3$ | 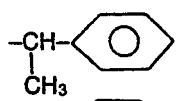 | $^1$H–NMR$^{*)}$ 5,7 ppm |
| 62 | $CH_3$ | 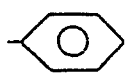 | 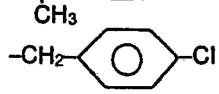 | F 120 |
| 63 | $CH_3$ | 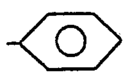 | $-CH_2-CN$ | F 135 |
| 64 | $CH_3$ | $CH_3$ | 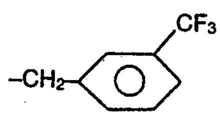 | F 60 |
| 65 | $CH_3$ | 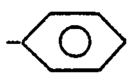 | $CH_3$ | F 98 |
| 66 | $CH_3$ | 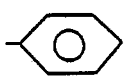 | $-CH_2-COOH$ | F 139 |
| 67 | $CH_3$ | 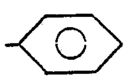 | 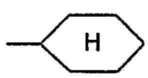 | $^1$H–NMR$^{*)}$ 4,5 ppm |
| 68 | $CH_3$ | H | 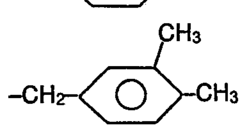 | $^1$H–MNR$^{*)}$ 5,5 ppm |
| 69 | $CH_3$ | H | 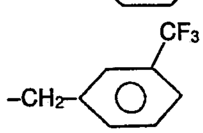 | $^1$H–NMR$^{*)}$ 5,5 ppm |
| 70 | $CH_3$ | H | 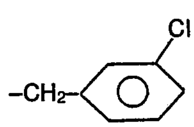 | $^1$H–NMR$^{*)}$ 5,4 ppm |

| Bsp.-Nr. | $R^1$ | $R^2$ | $R^3$ | Phys. Daten [F = Schmelzpunkt in °C] |
|---|---|---|---|---|
| 71 | $CH_3$ | H | 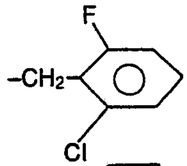 | F 133 |
| 72 | $CH_3$ | H | 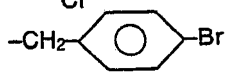 | $^{1}$–NMR*) 5,5 ppm |
| 73 | $CH_3$ | H | 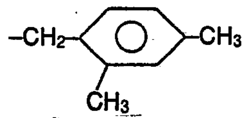 | $^{1}$H–NMR*) 5,5 ppm |
| 74 | $CH_3$ | $-CH_2-CN$ | 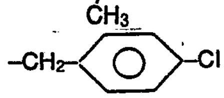 | $^{1}$H–NMR*) 4,6 und 5,5 ppm |
| 75 | $CH_3$ | H | 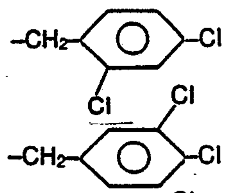 | F115–120 |
| 76 | $CH_3$ | $-CH_2-CN$ | | $^{1}$H–NMR*) 4,6 und 5,4 ppm |
| 77 | $CH_3$ | $-CH_2-CN$ | 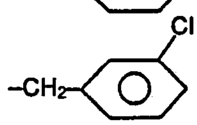 | $^{1}$H–NMR*) 4,6 und 5,5 ppm |
| 78 | $CH_3$ | $-CH_2-CH_2-CN$ | 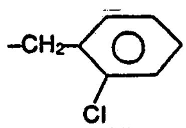 | F 58 |
| 79 | $CH_3$ | $-CH_2-CH_2-CN$ | 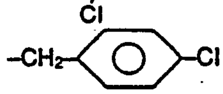 | F 101 |
| 80 | $CH_3$ | $-CH_2-CN$ | 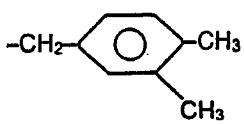 | $^{1}$H–NMR*) 4,6 und 5,5 ppm |
| 81 | $CH_3$ | $-CH_2-CN$ | 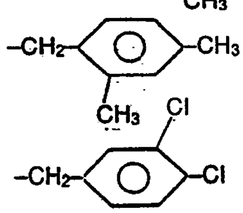 | $^{1}$H–NMR*) 4,6 und 5,5 ppm |
| 82 | $CH_3$ | $-CH_2-CH_2-CN$ | | F 120 |
| 83 | $CH_3$ | $-CH_2-CH_2-CN$ | 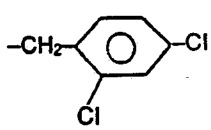 | F 106 |
| 84 | $CH_3$ | $-CH_2-CN$ | 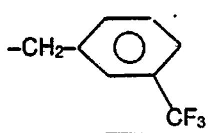 | $^{1}$H–NMR*) 4,6 und 5,5 ppm |
| 85 | $CH_3$ | $-CH_2-CN$ | 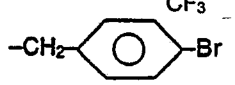 | $^{1}$H–NMR*) 4,6 und 5,5 ppm |
| 86 | $CH_3$ | $-CH_2-CN$ | 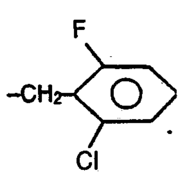 | $^{1}$H–NMR*) 4,6 und 5,7 ppm |

| Bsp.-Nr. | $R^1$ | $R^2$ | $R^3$ | Phys. Daten [F = Schmelzpunkt in °C] |
|---|---|---|---|---|
| 87 | $CH_3$ | $-CH_2-CN$ | 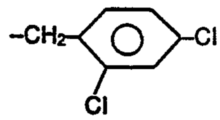 | $^1$H-NMR*) 4,6 und 5,5 ppm |
| 88 | $CH_3$ | $CH_3$ | 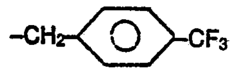 | $^1$H-NMR*) 5,5 ppm |
| 89 | $CH_3$ | $CH_3$ | 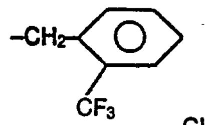 | F 72 |
| 90 | $CH_3$ | $CH_3$ | 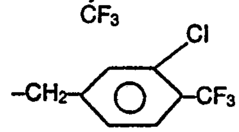 | F 87 |
| 91 | $CH_3$ | $CH_3$ | 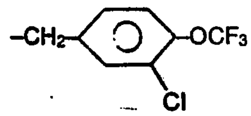 | F 63–67 |
| 92 | $CH_3$ | $CH_3$ | 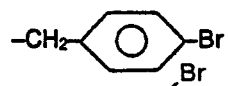 | F 103 |
| 93 | $CH_3$ | $CH_3$ | 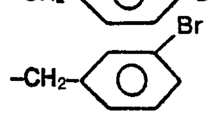 | F 90 |
| 94 | $CH_3$ | $CH_3$ | 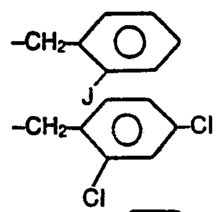 | F 103–106 |
| 95 | $CH_3$ | $CH_3$ | | F 111 |
| 96 | $CH_3$ | $CH_3$ | 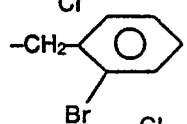 | F 101 |
| 97 | $CH_3$ | $CH_3$ | 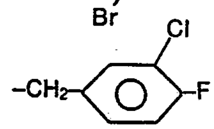 | F 95 |
| 98 | $CH_3$ | $CH_3$ | 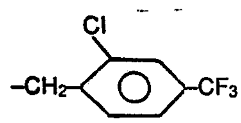 | F 68–72 |
| 99 | $CH_3$ | $CH_3$ | 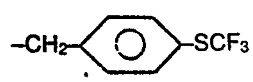 | $^1$H-NMR*) 5,55 ppm |
| 100 | $CH_3$ | $-CH_2-CH_2-CN$ | 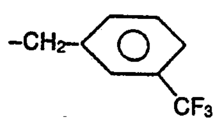 | F 79 |
| 101 | $CH_3$ | $-CH_2-CH_2-CN$ | 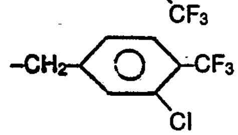 | F 70–75 |
| 102 | $CH_3$ | $CH_3$ | 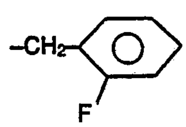 | F 64 |

| Bsp.-Nr. | $R^1$ | $R^2$ | $R^3$ | Phys. Daten [F = Schmelzpunkt in °C] |
|---|---|---|---|---|
| 103 | $CH_3$ | $CH_3$ | 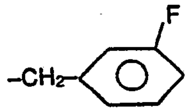 | F 75–80 |
| 104 | $CH_3$ | $CH_3$ | 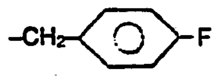 | $^1$H-NMR*) 5,45 ppm |
| 105 | $CH_3$ | H | 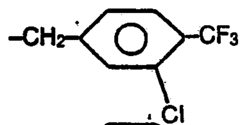 | F 123 |
| 106 | $CH_3$ | $CH_3$ | 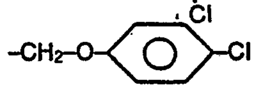 | F 88 |
| 107 | $CH_3$ | $-CH_2-CH_2-CN$ | 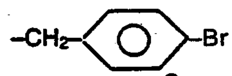 | F 90 |
| 108 | $CH_3$ | $CH_3$ | 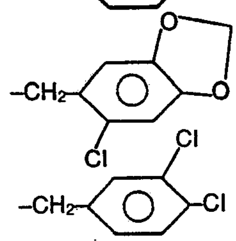 | F 125 |
| 109 | $C_2H_5$ | $CH_3$ | | H-NMR*) 5.5 ppm |
| 110 | $CH_3$ | $CH_3$ | 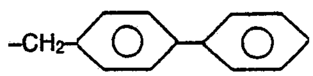 | $^1$H-NMR*) 5.45 ppm |
| 111 | $CH_3$ | $CH_3$ | 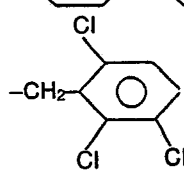 | F 130 |
| 112 | $CH_3-(CH_2)_2-$ | $CH_3$ | 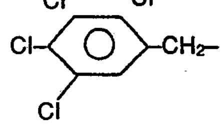 | F 56 |
| 113 | $CH_3-(CH_2)_3-$ | H | 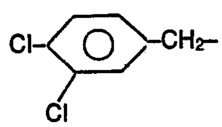 | F 112 |
| 114 | $C_2H_5$ | $CH_3$ | 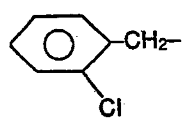 | F 58 |
| 115 | $CH_3-(CH_2)_2-$ | $CH_3$ | 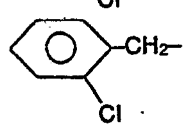 | F 58 |
| 116 | $CH_3-(CH_2)_2-$ | H | 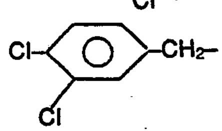 | F 104 |
| 117 | $C_2H_5-$ | $CH_3$ | 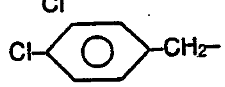 | F 94 |
| 118 | $CH_3-(CH_2)_3-$ | $CH_3$ | 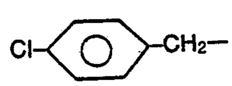 | F 114 |

| Bsp.-Nr. | $R^1$ | $R^2$ | $R^3$ | Phys. Daten [F = Schmelzpunkt in °C] |
|---|---|---|---|---|
| 119 | $CH_3-(CH_2)_3-$ | $CH_3$ | 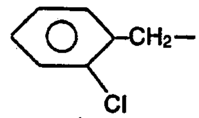 | F 42 |
| 120 | $C_2H_5$ | H | 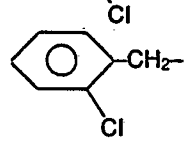 | F 103 |
| 121 | $CH_3-(CH_2)_2-$ | H | 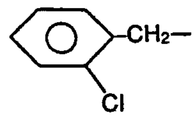 | F 78 |
| 122 | $CH_3-(CH_2)_3-$ | H | 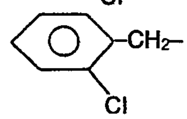 | F 93 |
| 123 | $CH_3$ | $CH_3$ | 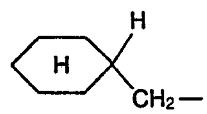 | Öl $^1$H-NMR*) 4.3 ppm |
| 124 | $CH_3-(CH_2)_3-$ | $NC-CH_2-$ | 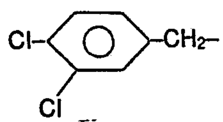 | Öl $^1$H-NMR*) 5.35; 4,6 ppm |
| 125 | $CH_3-(CH_2)_3-$ | $NC-CH_2-$ | 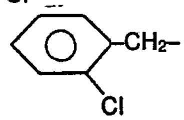 | F 55 |
| 126 | $C_2H_5$ | $NC-CH_2-$ | 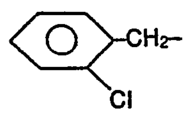 | Öl $^1$H-NMR*) 5.6; 4.6 ppm |
| 127 | $CH_3-(CH_2)_2-$ | $NC-CH_2-$ | 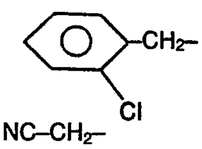 | Öl $^1$H-NMR*) 5.6; 4.5 ppm |
| 128 | $CH_3$ | 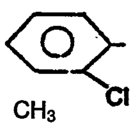 | $NC-CH_2-$ | Öl $^1$H-NMR*) 5.0 ppm |
| 129 | $(CH_3)_3C-$ | $CH_3$ | $NC-CH_2-$ | Öl $^1$H-NMR*) 5.0 ppm |
| 130 | $CH_3-(CH_2)_3-$ | $CH_3$ | 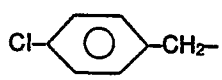 | F 40 |
| 131 | $CH_3$ | 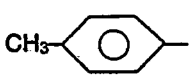 | $NC-CH_2-$ | F 91 |
| 132 | $CH_3$ | $(CH_3)_3C-$ | $NC-CH_2-$ | Öl $^1$H-NMR*) 5.0 ppm |
| 133 | $CH_3$ | H | 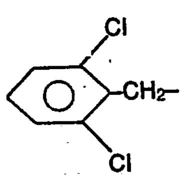 | F 144 |
| 134 | $CH_3$ | H | 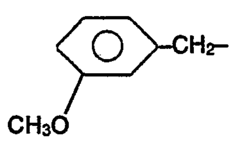 | F 86 |

| Bsp.-Nr. | $R^1$ | $R^2$ | $R^3$ | Phys. Daten [F = Schmelzpunkt in °C] |
|---|---|---|---|---|
| 135 | $CH_3$ | H | 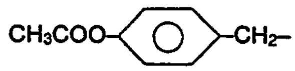 | F 109 |
| 136 | $CH_3$ | H | 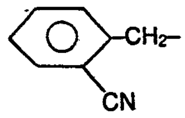 | F 147 |
| 137 | $CH_3$ | H | 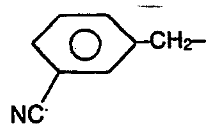 | F 140 |
| 138 | $CH_3$ | H | 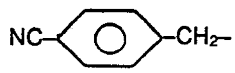 | F 169 |
| 139 | $CH_3$ | $NC–CH_2–$ | 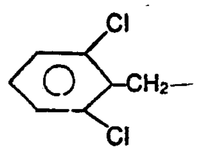 | Öl $^1$H–NMR*) 5.8 ppm |
| 140 | $CH_3$ | $HC \equiv C–CH_2–$ | 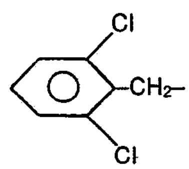 | Öl $^1$H–NMR*) 5.75 ppm |
| 141 | $CH_3$ | $H_2C=CH–CH_2–$ | 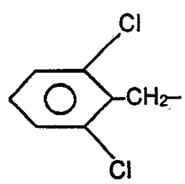 | F 80 |
| 142 | $CH_3$ | $NC–CH_2–$ | 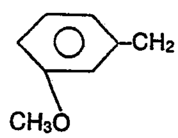 | Öl $^1$H–NMR*) 5.5 ppm |
| 143 | $CH_3$ | $NC–CH_2–$ | 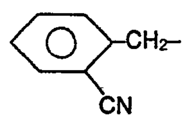 | Öl $^1$H–NMR*) 5.65 ppm |
| 144 | $CH_3$ | $NC–CH_2–$ | 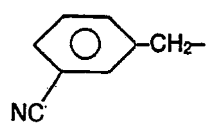 | Öl $^1$H–NMR*) 5.5 ppm |
| 145 | $CH_3$ | $NC–CH_2–$ | 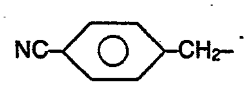 | F 122 |
| 146 | $CH_3$ | $H_2C=CH–CH_2–$ | 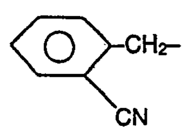 | F 83 |
| 147 | $CH_3$ | H | 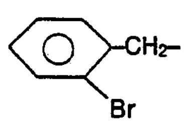 | F 126 |

| Bsp.-Nr. | $R^1$ | $R^2$ | $R^3$ | Phys. Daten [F = Schmelzpunkt in °C] |
|---|---|---|---|---|
| 148 | $CH_3$ | H | 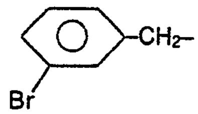 | F 74 |
| 149 | $CH_3$ | $H_2C=CH-CH_2-$ | 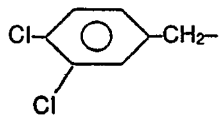 | Öl $^1$H-NMR*) 5.4 ppm |
| 150 | $CH_3$ | $HC\equiv C-CH_2-$ | 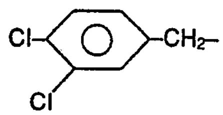 | Öl $^1$H-NMR*) 5.4 ppm |
| 151 | $CH_3$ | $H_2C=CH-CH_2$ | 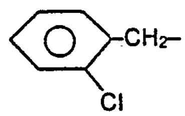 | F 55 |
| 152 | $CH_3$ | $HC\equiv C-CH_2-$ | 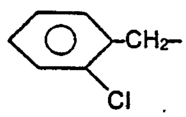 | F 77 |
| 153 | $CH_3$ | $NC-CH_2-$ | 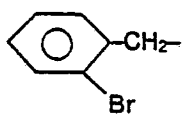 | F 116 |
| 154 | $CH_3$ | $NC-CH_2-$ | 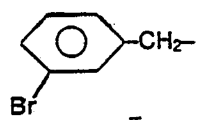 | Öl $^1$H-NMR*) 5.5 ppm |
| 155 | $CH_3$ | H | 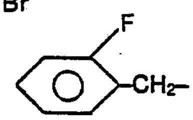 | F 72 |
| 156 | $CH_3$ | H | 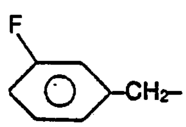 | F 94 |
| 157 | $CH_3$ | H | 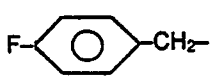 | F 115 |
| 158 | $CH_3$ | H | 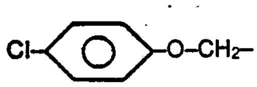 | F 98 |
| 159 | $CH_3$ | $HO-CH_2-CH_2-$ | 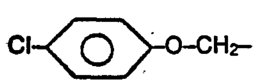 | F 74 |
| 160 | $CH_3$ | $HO-CH_2-CH_2-$ | 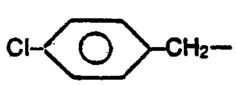 | Öl $^1$H-NMR*) 5.4 ppm |
| 161 | $CH_3$ | $HO-CH_2-CH_2-$ | 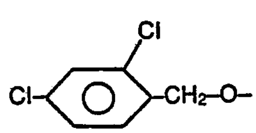 | F 102 |

| Bsp.-Nr. | $R^1$ | $R^2$ | $R^3$ | Phys. Daten [F = Schmelzpunkt in °C] |
|---|---|---|---|---|
| 162 | $CH_3$ | $HO-CH_2-CH_2-$ | 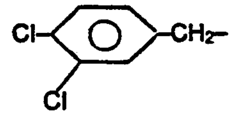 | F 99 |
| 163 | $CH_3$ | $HO-CH_2-CH_2-$ | 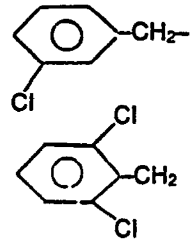 | Öl $^1H$-NMR*) 5.4 ppm |
| 164 | $CH_3$ | $HO-CH_2-CH_2-$ | | F 87 |
| 165 | $CH_3$ | $NC-CH_2-$ | 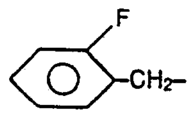 | F 83 |
| 166 | $CH_3$ | $CH_2=CH-CH_2$ | 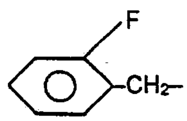 | Öl $^1H$-NMR*) 5.5 ppm |
| 167 | $CH_3$ | $NC-CH_2-$ | 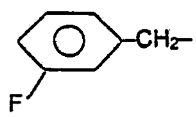 | F 60 |
| 168 | $CH_3$ | $NC-CH_2-$ | 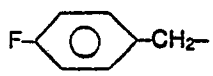 | F 73 |
| 169 | $CH_3$ | $NC-CH_2-$ | 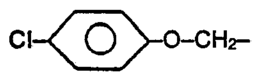 | Öl $^1H$-NMR*) 5.9 ppm |
| 170 | $CH_3$ | $H_2C=CH-CH_2-$ | 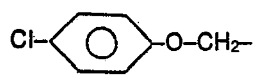 | Öl $^1H$-NMR*) 5.9 ppm |
| 171 | $CH_3$ | $HO-CH_2-CH_2-$ | 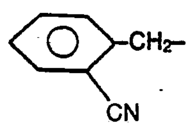 | F 86 |
| 172 | $CH_3$ | $HO-CH_2-CH_2-$ | 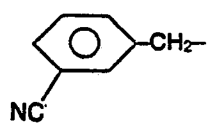 | F 78 |
| 173 | $CH_3$ | $HO-CH_2-CH_2-$ | 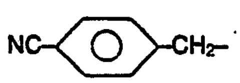 | F 130 |
| 174 | $CH_3$ | $CH_3$ | 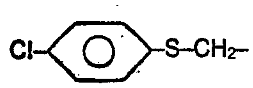 | F 72 |
| 175 | $CH_3$ | $NC-CH_2-CH_2-$ | 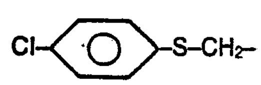 | Öl $^1H$-NMR*) 5.75 ppm |
| 176 | $CH_3$ | $C_2H_5-O-CO-CH_2-$ | 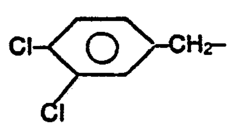 | F 83 |
| 177 | $CH_3$ | $HC\equiv C-CH_2-$ | 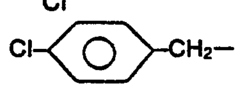 | F 75 |

| Bsp.-Nr. | $R^1$ | $R^2$ | $R^3$ | Phys. Daten [F = Schmelzpunkt in °C] |
|---|---|---|---|---|
| 178 | $CH_3$ | $C_2H_5$–OCO–$CH_2$– | 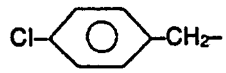 | Öl $^1$H–NMR*) 5.4 ppm |
| 179 | $CH_3$ | HC≡C–$CH_2$– | 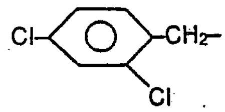 | F 97 |
| 180 | $CH_3$ | $C_2H_5$–O–CO–$CH_2$– | 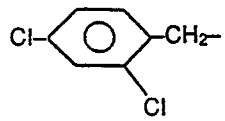 | F 72 |
| 181 | $CH_3$ | $CH_3$ | 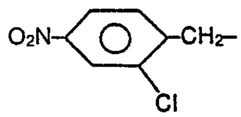 | F 118–120 |
| 182 | $CH_3$ | $CH_3$ | 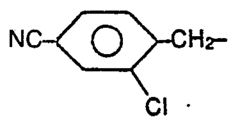 | F 165–175 |
| 183 | $CH_3$ | 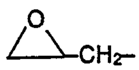 | 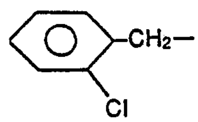 | F 68 |
| 184 | $CH_3$ | 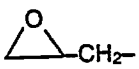 | 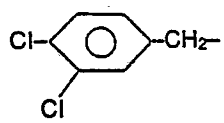 | F 75 |
| 185 | $CH_3$ | 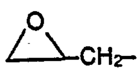 | 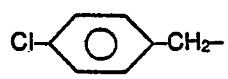 | F 60 |
| 186 | $CH_3$ | 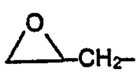 | 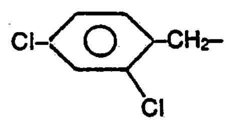 | F 85 |
| 187 | $CH_3$ | $H_2N$–CO–$CH_2$– | 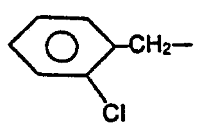 | F 211 |
| 188 | $CH_3$ | $(CH_3)_2N$–CO–$CH_2$– | 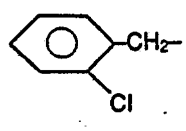 | Öl $^1$H-NMR*) 5.6 ppm |
| 189 | $CH_3$ | $CH_3$–NH–CO–$CH_2$– | 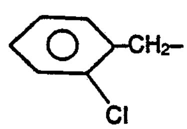 | F 149 |
| 190 | H | $CH_3$ | 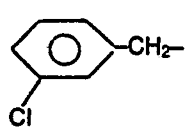 | Öl $^1$H–NMR*) 5.4 ppm |

| Bsp.-Nr. | $R^1$ | $R^2$ | $R^3$ | Phys. Daten [F = Schmelzpunkt in °C] |
|---|---|---|---|---|
| 191 | H | $CH_3$ | 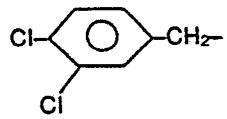 | F 92 |
| 192 | H | H | 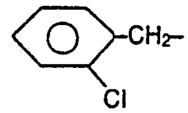 | F 131 |
| 193 | H | H | 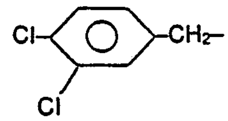 | F 136 |
| 194 | H | $CH_3$ | 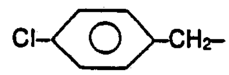 | F 76 |
| 195 | H | $CH_3$ | 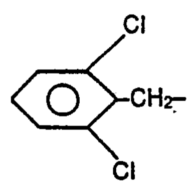 | F 85 |
| 196 | H | $CH_3$ | 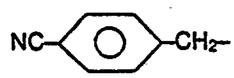 | F 81 |
| 197 | H | $CH_3$ | 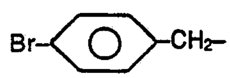 | F 94 |
| 198 | H | $CH_3$ | 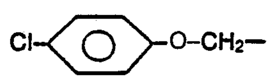 | F 74 |
| 199 | H | $NC-CH_2-CH_2-$ | 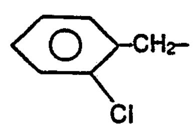 | Öl $^{1}H$-NMR*) 6.0 ppm |
| 200 | H | $NC-CH_2-CH_2-$ | 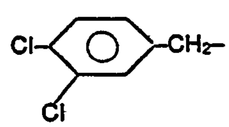 | Öl $^{1}H$-NMR*) 5.3 ppm |
| 201 | H | $CH_3$ | 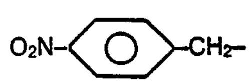 | F 130 |
| 202 | H | $CH_3$ | 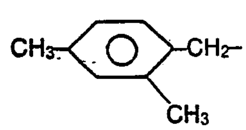 | Öl $^{1}H$-NMR*) 5.3 ppm |
| 203 | H | $CH_3$ | 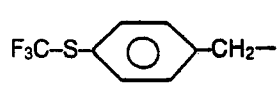 | Öl $^{1}H$-NMR*) 5.5 ppm |

| Bsp.-Nr. | $R^1$ | $R^2$ | $R^3$ | Phys. Daten [F = Schmelzpunkt in °C] |
|---|---|---|---|---|
| 204 | H | $CH_3$ | 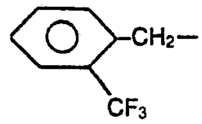 | F 49–51 |
| 205 | H | $CH_3$ | 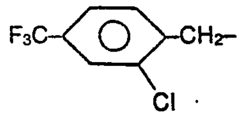 | F 97 |
| 206 | H | $CH_3$ | 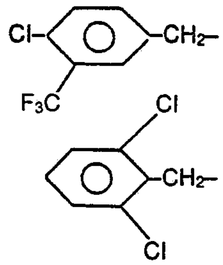 | Öl $^1$H–NMR*) 5.45 ppm |
| 207 | H | $NC-CH_2-CH_2-$ | | F 116–119 |
| 208 | H | $NC-CH_2-CH_2-$ | 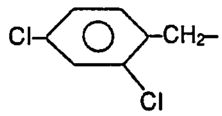 | F 118–120 |
| 209 | H | $CH_3$ | 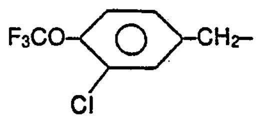 | Öl $^1$H–NMR*) 5.45 ppm |
| 210 | H | $NC-CH_2-CH_2-$ | 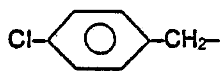 | Öl $^1$H–NMR*) 5.6 ppm |
| 211 | H | $NC-CH_2-CH_2-$ | 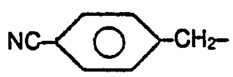 | Öl $^1$H–NMR*) 5.5 ppm |
| 212 | H | $NC-CH_2-CH_2-$ | 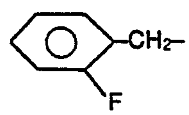 | F 98–100 |
| 213 | H | $NC-CH_2-CH_2-$ | 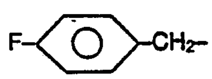 | Öl $^1$H–NMR*) 5.5 ppm |
| 214 | H | $HO-CH_2-CH_2-$ | 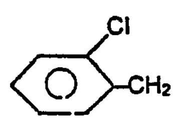 | Öl $^1$H–NMR*) 5.6 ppm |
| 215 | H | $HO-CH_2-CH_2-$ | 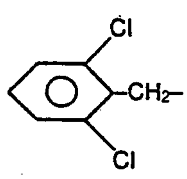 | F 105–108 |
| 216 | H | $HO-CH_2-CH_2-$ | 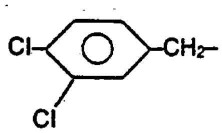 | Öl $^1$H–NMR*) 5.4 ppm |

| Bsp.-Nr. | $R^1$ | $R^2$ | $R^3$ | Phys. Daten [F = Schmelzpunkt in °C] |
|---|---|---|---|---|
| 217 | H | $NC-CH_2-CH_2-$ | 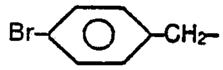 | F 112–114 |
| 218 | H | $HO-CH_2-CH_2-$ | 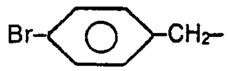 | Öl $^1$H-NMR*) 5.4 ppm |
| 219 | H | $CH_3$ | 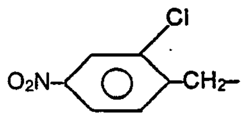 | F 118 |
| 220 | H | $HO-CH_2-CH_2-$ | 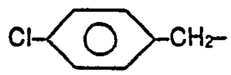 | Öl $^1$H-NMR*) 5.45 ppm |
| 221 | H | $HO-CH_2-CH_2-$ | 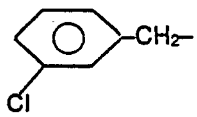 | Öl $^1$H-NMR*) 5.45 ppm |
| 222 | H | $HO-CH_2-CH_2-$ | 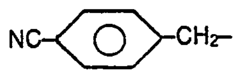 | F 75 |
| 223 | H | $HO-CH_2-CH_2-$ | 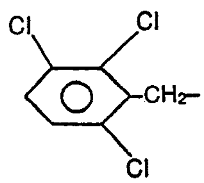 | F 90 |
| 224 | H | $HO-CH_2-CH_2-$ | 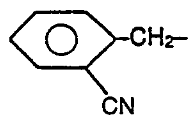 | Öl $^1$H-NMR*) 5.6 ppm |
| 225 | H | $NC-CH_2-CH_2-$ | 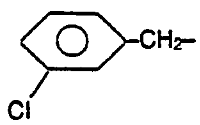 | Öl $^1$H-NMR*) 5.4 ppm |
| 226 | H | $NC-CH_2-CH_2-$ | 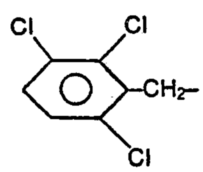 | F 118–119 |
| 227 | H | $NC-CH_2-CH_2-$ | 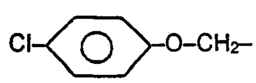 | F 94 |
| 228 | H | 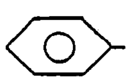 | 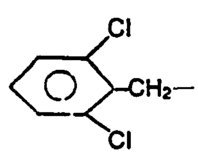 | F 98–100 |
| 229 | H | 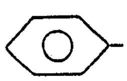 | 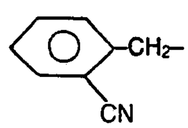 | F 102–104 |
| 230 | H | 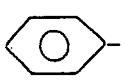 | 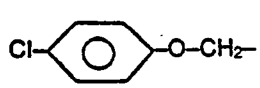 | F 137–139 |

| Bsp.-Nr. | $R^1$ | $R^2$ | $R^3$ | Phys. Daten [F = Schmelzpunkt in °C] |
|---|---|---|---|---|
| 231 | H | H | 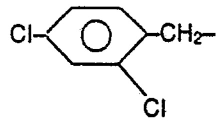 | F 105 |
| 232 | H | H | 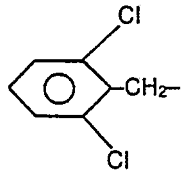 | F 157–159 |
| 233 | H | H | 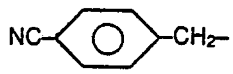 | F 144–146 |
| 234 | H | $NC-CH_2-$ | 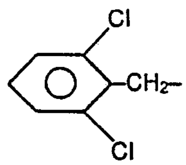 | Öl $^1$H–NMR*) 5.8 ppm |
| 235 | H | $NC-CH_2-$ | 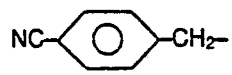 | F 110 |
| 236 | H | $CH_3$ | 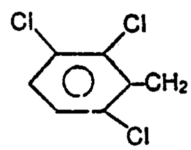 | F 98 |
| 237 | H | $C_2H_5-O-CO-CH_2-$ | 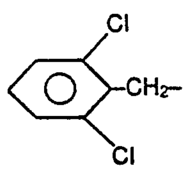 | F 124 |
| 238 | H | $C_2H_5-O-CO-CH_2-$ | 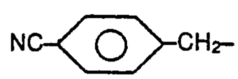 | Öl $^1$H–NMR*) 5.35 ppm |
| 239 | H | $NC-CH_2-$ | 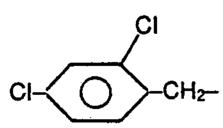 | Öl $^1$H–NMR*) 5.5 ppm |
| 240 | H | $C_2H_5-O-CO-CH_2-$ | 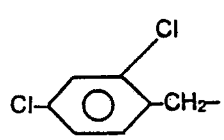 | F 74 |
| 241 | H | $NC-CH_2-$ | 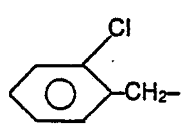 | Öl $^1$H–NMR*) 5.66 ppm |
| 242 | H | $C_2H_5-O-CO-CH_2-$ | 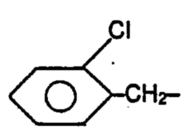 | F 64 |

| Bsp.-Nr. | $R^1$ | $R^2$ | $R^3$ | Phys. Daten [F = Schmelzpunkt in °C] |
|---|---|---|---|---|
| 243 | H | $NC-CH_2-$ | 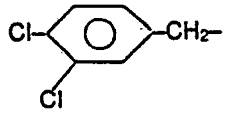 | F 103–105 |
| 244 | H | $C_2H_5-O-CO-CH_2-$ | 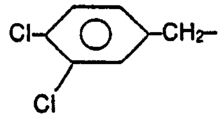 | F 74–75 |
| 245 | H | H | 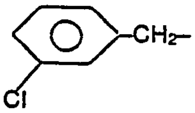 | F 131 |

*) Die $^1H$-NMR-Spektren wurden aufgenommen in $CDCl_3$ mit Tetramethylsilan als innerem Standard. Angegeben sind in der Regel die chemischen Verschiebungen als δ-Werte für die Gruppierung $>=N-O-CH_2-$.

Anwendungsbeispiele

In den folgenden Anwendungsbeispielen wurden die nachstehend aufgeführten Verbindungen als Vergleichssubstanzen eingesetzt:

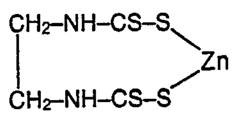 (A)

Zink-ethylen-1,2-bis-(dithiocarbamat)

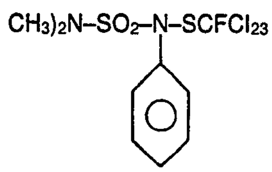 (B)

N,N-Dimethyl-N'-(fluordichlormethylmercapto)-N'-phenylsulfamid.

Beispiel A

Phytophthora-Test (Tomate)/protektiv

Lösungsmittel: 4,7 Gewichtsteile Aceton

Emulgator: 0,3 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wässrigen Sporensuspension von Phytophthora infestans inokuliert.

Die Pflanzen werden in einer Inkubationskabine mit 100% relativer Luftfeuchtigkeit und ca. 20°C aufgestellt.

3 Tage nach der Inokulation erfolgt die Auswertung.

Eine deutliche Überlegenheit in der Wirksamkeit gegenüber dem Stand der Technik zeigt in diesem Test z.B. die Verbindung gemäss dem Herstellungsbeispiel: 9.

Beispiel B

Pyricularia-Test (Reis)/protektiv

Lösungsmittel: 12,5 Gewichtsteile Aceton

Emulgator: 0,3 Gewichtsteile Alkylarylpolyglykolether

Zur Herstellung einer zweckmässigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wässrigen Sporensuspension von Pyricularia oryzae inokuliert. Anschliessend werden die Pflanzen in einem Gewächshaus bei 100% rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäss den Herstellungsbeispielen: 1, 12, 14, 163, 192, 193, 200, 201, 206, 212, 213, 214 und 215.

Beispiel C

Pyricularia-Test (Reis)/systemisch

Lösungsmittel: 12,5 Gewichtsteile Aceton

Emulgator: 0,3 Gewichtsteile Alkylarylpolyglykolether

Zur Herstellung einer zweckmässigen Wirkstoffzubereitung vermischt man 1 Gewichtsteil Wirkstoff mit der angegebenen Menge Lösungsmittel

und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wässrigen Sporensuspension von Pyricularia oryzae inokuliert. Danach verbleiben die Pflanzen in einem Gewächshaus bei einer Temperatur von 25 °C und einer rel. Luftfeuchtigkeit von 100% bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäss den Herstellungsbeispielen: 2, 11 und 16.

Beispiel D
Cochliobolus sativus-Test (Gerste)/protektiv
Lösungsmittel: 100 Gewichtsteile Dimethylformamid
Emulgator: 0,25 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20 °C und 100% rel. Luftfeuchtigkeit in einer Inkubationskabine.

Die Pflanzen werden in einem Gewächshaus bei einer Temperatur von ca. 20 °C und einer relativen Luftfeuchtigkeit von ca. 80% aufgestellt.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäss den Herstellungsbeispielen: 1, 2, 9, 15, 16, 17, 18, 19 und 21.

Beispiel E
Botrytis-Test (Bohne)/protektiv
Lösungsmittel: 4,7 Gewichtsteile Aceton
Emulgator: 0,3 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Grösse der Befallsflecken auf den Blättern ausgewertet.

Eine deutliche Überlegenheit in Wirksamkeit gegenüber dem Stand der Technik zeigen in diesem Test z.B. die Verbindungen gemäss den Herstellungsbeispielen: 191, 192, 193, 194, 197, 201, 203, 204, 205, 206.

Beispiel F
Leptosphaeria nodorum-Test (Weizen)/protektiv
Lösungsmittel: 100 Gewichtsteile Dimethylformamid
Emulgator: 0,25 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20 °C und 100% relativer Luftfeuchtigkeit in einer Inkubationskabine.

Die Pflanzen werden in einem Gewächshaus bei einer Temperatur von ca. 15 °C und einer relativen Luftfeuchtigkeit von ca. 80% aufgestellt.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäss den Herstellungsbeispielen: 190, 191, 192, 193.

**Patentansprüche**

1. Substituierte Pyrazolin-5-one der Formel (I)

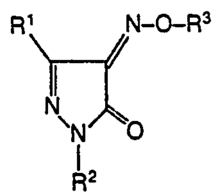

(I)

in welcher

$R^1$ für Wasserstoff oder für geradkettiges oder verzweigtes Alkyl mit 1 bis 8 Kohlenstoffatomen steht,

$R^2$ für Wasserstoff, für jeweils geradkettiges oder verzweigtes Alkyl, Alkenyl, Alkinyl, Cyanalkyl, Hydroxyalkyl, Alkoxyalkyl, Hydroxycarbonylalkyl, Alkoxycarbonylalkyl, Aminocarbonylalkyl, Alkylaminocarbonylalkyl oder Dialkylaminocarbonylalkyl mit jeweils bis zu 8 Kohlenstoffatomen in den einzelnen Alkyl- bzw. Alkenyl- oder Alkinylteilen, für Oxiranylalkyl mit 1 bis 4 Kohlenstoffatomen in geradkettigem oder verzweigtem Alkylteil, für geradkettiges oder verzweigtes, gegebenenfalls einfach oder mehrfach, gleich oder verschieden substituiertes Aralkyl mit 1 bis 4 Kohlenstoffatomen im Alkylteil und 6 bis 10 Kohlenstoffatomen im Arylteil oder für gegebenenfalls einfach oder mehrfach, gleich oder verschieden substituiertes Aryl mit 6 bis 10 Kohlenstoffatomen steht, wobei als Substituenten jeweils in Frage kommen: Halogen, Cyano, Nitro, Hydroxy, jeweils geradkettiges oder verzweigtes Alkyl, Alkoxy, Dioxyalkylen, Alkylcarbonyloxy oder Alkylthio mit jeweils bis zu 4 Kohlenstoffatomen, geradkettiges oder verzweigtes Halogenalkyl, Halogenalkoxy oder Halogenalkylthio mit jeweils bis zu 4 Kohlenstoffatomen und bis zu 9 gleichen oder verschiedenen Halogenatomen oder Phenyl und

$R^3$ für geradkettiges oder verzweigtes Alkyl mit 1 bis 20 Kohlenstoffatomen, für jeweils geradkettiges oder verzweigtes Alkenyl, Alkinyl, Cyanalkyl, Hydroxyalkyl, Alkoxyalkyl, Hydroxycarbonylalkyl oder Alkoxycarbonylalkyl mit jeweils bis zu 8 Kohlenstoffatomen in den einzelnen Alkyl- bzw. Alkenyl- oder Alkinylteilen, für Cycloalkyl mit 3 bis 7 Kohlenstoffatomen, Cycloalkylalkyl mit 3 bis 6 Kohlenstoffatomen im Cycloalkylteil und 1 oder 2 Kohlenstoffatomen im Alkylteil oder für jeweils gegebenenfalls einfach oder mehrfach, gleich oder verschieden substituiertes Arylalkyl, Aryloxyalkyl oder Arylthioalkyl mit 1 bis 4 Kohlenstoffatomen in den einzelnen Alkylteilen und 6 bis 10 Kohlenstoffatomen in den Arylteilen steht, wobei als Substituenten die bei $R^2$ genannten in Frage kommen, wobei jedoch $R^3$ nur dann für Methyl oder Ethyl steht, wenn $R^1$ und/oder $R^2$ nicht gleichzeitig für Wasserstoff oder Methyl stehen, weiterhin ist ausgenommen die Verbindung 1-Phenyl-3-methyl-4-benzyloximino-5-pyrazolon.

2. Substituierte Pyrazolin-5-one gemäss Anspruch 1, wobei in der Formel (I)

$R^1$ für Wasserstoff, Methyl, Ethyl, n- oder i-Propyl und n-, i-, s- oder t-Butyl steht,

$R^2$ für Wasserstoff, Methyl, Ethyl, n- oder i-Propyl, n-, i-, s- oder t-Butyl, Allyl, Butenyl, Propargyl, Cyanmethyl, Cyanethyl, Hydroxymethyl, Hydroxyethyl, Methoxymethyl, Methoxyethyl, Ethoxymethyl, Ethoxyethyl, Hydroxycarbonylmethyl, Hydroxycarbonylethyl, Methoxycarbonylmethyl, Methoxycarbonylethyl, Ethoxycarbonylmethyl, Ethoxycarbonylethyl, Aminocarbonylmethyl, Methylaminocarbonylmethyl, Ethylaminocarbonylethyl, Ethylaminocarbonylmethyl, Dimethylaminocarbonylmethyl, Diethylaminocarbonylmethyl, Aminocarbonylethyl, Oxiranylmethyl, Oxiranylethyl oder für jeweils gegebenenfalls ein- bis dreifach, gleich oder verschieden substituiertes Phenyl oder Benzyl steht, wobei als Substituenten in Frage kommen: Fluor, Chlor, Brom, Iod, Cyano, Nitro, Hydroxy, Methyl, Ethyl, n- oder i-Propyl, n-, i-, s- oder t-Butyl, Methoxy, Ethoxy, Dioxymethylen, Dioxyethylen, Methylthio, Ethylthio, Acetoxy oder Propionyloxy, Chlormethyl, Dichlormethyl, Trichlormethyl, Trifluormethyl, Dichlorfluormethyl, Chlordifluormethyl, Trifluormethoxy, Trifluormethylthio oder Phenyl und

$R^3$ für Methyl, Ethyl, n- oder i-Propyl, n- oder i-Butyl, n- oder i-Pentyl, n- oder i-Hexyl, n- oder i-Heptyl, n- oder i-Octyl, n- oder i-Nonyl, n- oder i-Decyl, n- oder i-Dodecyl, n- oder i-Hexadecyl, Allyl, Butenyl, Propargyl, Cyanmethyl, Cyanethyl, Hydroxymethyl, Hydroxyethyl, Methoxymethyl, Methoxyethyl, Ethoxymethyl, Ethoxyethyl, Hydroxycarbonylmethyl, Hydroxycarbonylethyl, Methoxycarbonylmethyl, Methoxycarbonylethyl, Ethoxycarbonylmethyl, Ethoxycarbonylethyl, Cyclopropyl, Cyclopentyl, Cyclohexyl, Cyclohexylmethyl oder für einen gegebenenfalls ein- bis dreifach, gleich oder verschieden substituierten Rest der Formel

$Ar-(Z)_m-CH_2)_n-$, 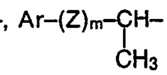

oder

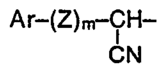

steht, wobei

Ar jeweils für Phenyl oder Naphthyl steht,
Z jeweils für Sauerstoff oder Schwefel steht,
m jeweils für 0 oder 1 steht und
n für 1, 2 oder 3 steht,

und wobei als Substituenten die bei $R^2$ genannten in Frage kommen, mit der Einschränkung, dass $R^3$ nur dann für Methyl oder Ethyl steht, wenn $R^1$ und $R^2$ nicht gleichzeitig für Wasserstoff oder Methyl stehen, weiterhin ist ausgenommen die Verbindung 1-Phenyl-3-methyl-4-benzyloximino-5-pyrazolon.

3. Verfahren zur Herstellung von substituierten Pyrazolin-5-onen der Formel (I)

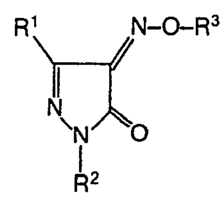

(I)

in welcher

$R^1$ für Wasserstoff oder für geradkettiges oder verzweigtes Alkyl mit 1 bis 8 Kohlenstoffatomen steht,

$R^2$ für Wasserstoff, für jeweils geradkettiges oder verzweigtes Alkyl, Alkenyl, Alkinyl, Cyanalkyl, Hydroxyalkyl, Alkoxyalkyl, Hydroxycarbonylalkyl, Alkoxycarbonylalkyl, Aminocarbonylalkyl, Alkylaminocarbonylalkyl oder Dialkylaminocarbonylalkyl mit jeweils bis zu 8 Kohlenstoffatomen in den einzelnen Alkyl- bzw. Alkenyl- oder Alkinylteilen, für Oxiranylalkyl mit 1 bis 4 Kohlenstoffatomen in geradkettigem oder verzweigtem Alkylteil, für geradkettiges oder verzweigtes, gegebenenfalls einfach oder mehrfach, gleich oder verschieden substituiertes Aralkyl mit 1 bis 4 Kohlenstoffatomen im Alkylteil und 6 bis 10 Kohlenstoffatomen im Arylteil oder für gegebenenfalls einfach oder mehrfach, gleich oder verschieden substituiertes Aryl mit 6 bis 10 Kohlenstoffatomen steht, wobei als Substituenten jeweils in Frage kommen: Halogen, Cyano, Nitro, Hydroxy, jeweils geradkettiges oder verzweigtes Alkyl, Alkoxy, Dioxyalkylen, Alkylcarbonyloxy oder Alkylthio mit jeweils bis zu 4 Kohlenstoffatomen, geradkettiges oder verzweigtes Halogenalkyl, Halogenalkoxy oder Halogenalkylthio mit jeweils bis zu 4 Kohlenstoffatomen und bis zu 9 gleichen oder verschiedenen Halogenatomen oder Phenyl und

$R^3$ für geradkettiges oder verzweigtes Alkyl mit 1 bis 20 Kohlenstoffatomen, für jeweils geradkettiges oder verzweigtes Alkenyl, Alkinyl, Cyanalkyl, Hydroxyalkyl, Alkoxyalkyl, Hydroxycarbonylalkyl oder Alkoxycarbonylalkyl mit jeweils bis zu 8 Kohlenstoffatomen in den einzelnen Alkyl- bzw. Alkenyl- oder Alkinylteilen, für Cycloalkyl mit 3 bis 7 Kohlenstoffatomen, Cycloalkylalkyl mit 3 bis 6 Kohlenstoffatomen im Cycloalkylteil und 1 oder 2 Kohlenstoffatomen im Alkylteil oder für jeweils gegebenenfalls einfach oder mehrfach, gleich oder verschieden substituiertes Arylalkyl, Aryloxyalkyl oder Arylthioalkyl mit 1 bis 4 Kohlenstoffatomen in den einzelnen Alkylteilen und 6 bis 10 Kohlenstoffatomen in den Arylteilen steht, wobei als Substituenten die bei $R^2$ genannten in Frage kommen, wobei jedoch $R^3$ nur dann für Methyl oder Ethyl steht, wenn $R^1$ und/oder $R^2$ nicht gleichzeitig für Wasserstoff oder Methyl stehen, weiterhin ist ausgenommen die Verbindung 1-Phenyl-3-methyl-4-benzyloximino-5-pyrazolon, dadurch gekennzeichnet, dass man

(a) 4-Oximino-pyrazolin-5-one der Formel (II)

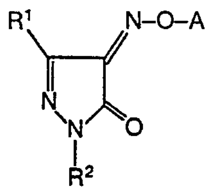

(II)

in welcher

$R^1$ und $R^2$ die oben angegebene Bedeutung haben und

A für Wasserstoff oder ein Alkalimetallkation steht,

mit Alkylierungsmitteln der Formel (III)

$$R^3\text{–}X \qquad (III)$$

in welcher

$R^3$ die oben angegebene Bedeutung hat und

X für eine elektronenanziehende Abgangsgruppe steht,

gegebenenfalls in Gegenwart eines Verdünnungsmittels, gegebenenfalls in Gegenwart eines Säurebindemittels und gegebenenfalls in Gegenwart eines Phasentransfer-Katalysators umsetzt, oder dass man

(b) die nach Verfahren (a) erhältlichen 4-Alkoximinopyrazolin-5-one der Formel (Ia)

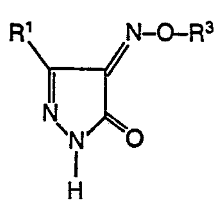

(Ia)

in welcher

$R^1$ und $R^3$ die oben angegebene Bedeutung haben

mit Alkylierungsmitteln der Formel (IV)

$$R^{2'}\text{–}Y \qquad (IV)$$

in welcher

$R^{2'}$ für Alkyl, Alkenyl, Alkinyl, Cyanalkyl, Hydroxyalkyl, Alkoxyalkyl, Hydroxycarbonylalkyl, Alkoxycarbonylalkyl oder für jeweils gegebenenfalls substituiertes Aralkyl oder Aryl steht und

Y für eine elektronenanziehende Abgangsgruppe steht,

gegebenenfalls in Gegenwart eines Verdünnungsmittels, gegebenenfalls in Gegenwart eines Säurebindemittels und gegebenenfalls in Gegenwart eines Phasentransfer-Katalysators umsetzt.

4. Schädlingsbekämpfungsmittel, gekennzeichnet durch einen Gehalt an mindestens einem substituierten Pyrazolin-5-on der Formel (I) gemäss den Ansprüchen 1 und 3.

5. Verwendung von substituierten Pyrazolin-5-onen der Formel (I) gemäss den Ansprüchen 1 und 3 zur Bekämpfung von Schädlingen.

6. Verfahren zur Bekämpfung von Schädlingen, dadurch gekennzeichnet, dass man substituierte Pyrazolin-5-one der Formel (I) gemäss den Ansprüchen 1 und 3 auf Schädlinge und/oder ihren Lebensraum einwirken lässt.

7. Verfahren zur Herstellung von Schädlingsbekämpfungsmitteln, dadurch gekennzeichnet, dass man substituierte Pyrazolin-5-one der Formel (I) gemäss den Ansprüchen 1 und 3 mit Streckmitteln und/oder oberflächenaktiven Mitteln vermischt.

**Claims**

1. Substituted pyrazolin-5-ones of the formula (I)

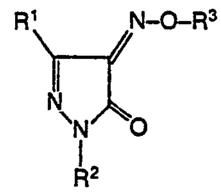

**(I)**

in which

$R^1$ represents hydrogen or straight-chain or branched alkyl with 1 to 8 carbon atoms,

$R^2$ represents hydrogen, or represents in each case straight-chain or branched alkyl, alkenyl, alkinyl, cyanoalkyl, hydroxyalkyl, alkoxyalkyl, hydroxycarbonylalkyl, alkoxycarbonylalkyl, aminocarbonylalkyl, alkylaminocarbonylalkyl or dialkylaminocarbonylalkyl with in each case up to 8 carbon atoms in the individual alkyl, alkenyl or alkinyl parts, represents oxiranylalkyl with 1 to 4 carbon atoms in the straight-chain or branched alkyl part, or represents straight-chain or branched aralkyl which has 1 to 4 carbon atoms in the alkyl part and 6 to 10 carbon atoms in the aryl part and is optionally monosubstituted or polysubstituted by identical or different substituents, or

represents aryl which has 6 to 10 carbon atoms and is optionally monosubstituted or polysubstituted by identical or different substituents, possible substituents in each case being: halogen, cyano, nitro, hydroxyl, in each case straight-chain or branched alkyl, alkoxy, dioxyalkylene, alkylcarbonyloxy and alkylthio with in each case up to 4 carbon atoms, straight-chain or branched halogenoalkyl, halogenoalkoxy and halogenoalkylthio with in each case up to 4 carbon atoms and up to 9 identical or different halogen atoms, or phenyl, and

$R^3$ represents straight-chain or branched alkyl with 1 to 20 carbon atoms, or represents in each case straight-chain or branched alkenyl, alkinyl, cyanoalkyl, hydroxyalkyl, alkoxyalkyl, hydroxycarbonylalkyl or alkoxycarbonylalkyl with in each case up to 8 carbon atoms in the individual alkyl, alkenyl or alkinyl parts, or represents cycloalkyl with 3 to 7 carbon atoms or cycloalkylalkyl with 3 to 6 carbon atoms in the cycloalkyl part and 1 or 2 carbon atoms in the alkyl part, or represents arylalkyl, aryloxyalkyl or arylthioalkyl with 1 to 4 carbon atoms in the individual alkyl parts and 6 to 10 carbon atoms in the aryl parts, and in each case optionally monosubstituted or polysubstituted by identical or different substituents, possible substituents being those mentioned for $R^2$, but wherein $R^3$ only represents methyl or ethyl if $R^1$ and/or $R^2$ do not simultaneously represent hydrogen or methyl, furthermore with the exception of the compound 1-phenyl-3-methyl-4-benzyloximino-5-pyrazolone.

2. Substituted pyrazolin-5-ones according to Claim 1 wherein, in formula (I),

$R^1$ represents hydrogen, methyl, ethyl, n- or i-propyl or n-, i-, s- or t-butyl,

$R^2$ represents hydrogen, methyl, ethyl, n- or i-propyl, n-, i-, s- or t-butyl, allyl, butenyl, propargyl, cyanomethyl, cyanoethyl, hydroxymethyl, hydroxyethyl, methoxymethyl, methoxyethyl, ethoxymethyl, ethoxyethyl, hydroxycarbonylmethyl, hydroxycarbonylethyl, methoxycarbonylmethyl, methoxycarbonylethyl, ethoxycarbonylmethyl or ethoxycarbonylethyl, aminocarbonylmethyl, methylaminocarbonylmethyl, ethylaminocarbonylethyl, ethylaminocarbonylmethyl, dimethyl, aminocarbonylmethyl, diethylaminocarbonylmethyl, aminocarbonylethyl, oxiranylmethyl and oxiranylethyl or represents phenyl or benzyl, in each case optionally mono-, di- or trisubstituted by identical or different substituents, possible substituents being: fluorine, chlorine, bromine, iodine, cyano, nitro, hydroxyl, methyl, ethyl-, n- or i-propyl, n-, i-, s- or t-butyl, methoxy, ethoxy, dioxymethylene, dioxyethylene, methylthio, ethylthio, acetoxy and propionyloxy, chloromethyl, dichloromethyl, trichloromethyl, trifluoromethyl, dichlorofluoromethyl, chlorodifluoromethyl, trifluoromethoxy, trifluoromethylthio or phenyl and

$R^3$ represents methyl, ethyl, n- or i-propyl, n- or i-butyl, n- or i-pentyl, n- or i-hexyl, n- or i-heptyl, n- or i-octyl, n- or i-nonyl, n- or i-decyl, n- or i-dodecyl, n- or i-hexadecyl, allyl, butenyl, propargyl, cyanomethyl, cyanoethyl, hydroxymethyl, hydroxyethyl, methoxymethyl, methoxyethyl, ethoxymethyl, ethoxyethyl, hydroxycarbonylmethyl, hydroxycarbonylethyl, methoxycarbonylmethyl, methoxycarbonylethyl, ethoxycarbonylmethyl, ethoxycarbonylethyl, cyclopropyl, cyclopentyl, cyclohexyl or cyclohexylmethyl, or represents a radical of the formula

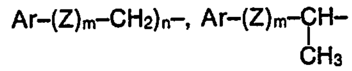

or

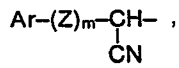

optionally mono-, di- or trisubstituted by identical or different substituents,

wherein

- Ar in each case represents phenyl or naphthyl,
- Z in each case represents oxygen or sulphur,
- m in each case represents 0 or 1 and
- n represents 1, 2 or 3,

possible substituents being those mentioned for $R^2$, with the restriction that $R^3$ only represents methyl or ethyl if $R^1$ and $R^2$ do not simultaneously represent hydrogen or methyl, furthermore with the exception of the compound 1-phenyl-3-methyl-4-benzyloximino-5-pyrazolone.

3. Process for the preparation of substituted pyrazolin-5-ones of the formula (I)

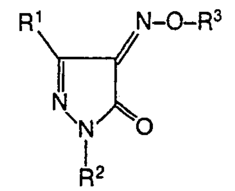

(I)

in which

$R^1$ represents hydrogen or straight-chain or branched alkyl with 1 to 8 carbon atoms,

$R^2$ represents hydrogen, or represents in each case straight-chain or branched alkyl, alkenyl, alkinyl, cyanoalkyl, hydoxyalkyl, alkoxyalkyl, hydroxycarbonylalkyl, alkoxycarbonylalkyl, aminocarbonylalkyl, alkylaminocarbonylalkyl or dialkylaminocarbonylalkyl with in each case up to 8 carbon atoms in the individual alkyl, alkenyl or alkinyl parts, represents oxiranylalkyl with 1 to 4 carbon atoms in the straight-chain or branched alkyl part, or represents straight-chain or branched aralkyl which has 1 to 4 carbon atoms in the alkyl part and 6 to 10 carbon atoms in the aryl part and is optionally monosubstituted or polysubstituted by identical or different substituents, or represents aryl which has 6 to 10 carbon atoms and is optionally monosubstituted or polysubstituted by identical or different substituents, possible substituents in each case being: halogen, cyano, nitro, hydroxyl, in each case straight-chain or branched alkyl, alkoxy, dioxyalkylene, alkylcarbonyloxy or alkylthio

with in each case up to 4 carbon atoms, straight-chain or branched halogenoalkyl, halogenoalkoxy and halogenoalkylthio with in each case up to 4 carbon atoms and up to 9 identical or different halogen atoms, or phenyl, and

$R^3$ represents straight-chain or branched alkyl with 1 to 20 carbon atoms, or represents in each case straight-chain or branched alkenyl, alkinyl, cyanoalkyl, hydroxyalkyl, alkoxyalkyl, hydroxycarbonylalkyl or alkoxycarbonylalkyl with in each case up to 8 carbon atoms in the individual alkyl, alkenyl or alkinyl parts, or represents cycloalkyl with 3 to 7 carbon atoms or cycloalkylalkyl with 3 to 6 carbon atoms in the cycloalkyl part and 1 or 2 carbon atoms in the alkyl part, or represents arylalkyl, aryloxyalkyl or arylthioalkyl with 1 to 4 carbon atoms in the individual alkyl parts and 6 to 10 carbon atoms in the aryl parts, and in each case optionally monosubstituted or polysubstituted by identical or different substituents, possible substituents being those mentioned for $R^2$, but wherein $R^3$ only represents methyl or ethyl if $R^1$ and/or $R^2$ do not simultaneously represent hydrogen or methyl, furthermore with the exception of the compound 1-phenyl-3-methyl-4-benzyloximine-5-pyrazolone, characterized in that

(a) 4-oximino-pyrazolin-5-ones of the formula (II)

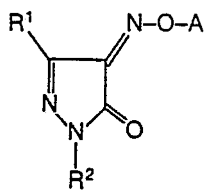

(II)

in which

$R^1$ and $R^2$ have the abovementioned meaning and

A represents hydrogen or an alkali metal cation, are reacted with alkylating agents of the formula (III)

$$R^3{-}X \qquad (III)$$

in which

$R^3$ has the abovementioned meaning and

X represents an electron-withdrawing leaving group,

if appropriate in the presence of a diluent, if appropriate in the presence of an acid-binding agent and if appropriate in the presence of a phase-transfer catalyst, or in which

(b) the 4-alkoximino-pyrazolin-5-ones obtainable by process (a), of the formula (Ia)

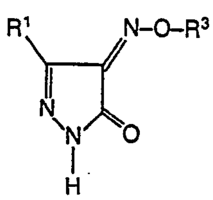

(Ia)

in which

$R^1$ and $R^3$ have the abovementioned meaning, are reacted with alkylating agents of the formula (IV)

$$R^{2'}{-}Y \qquad (IV)$$

in which

$R^{2'}$ represents alkyl, alkenyl, alkinyl, cyanoalkyl, hydroxyalkyl, alkoxyalkyl, hydroxycarbonylalkyl or alkoxycarbonylalkyl or in each case optionally substituted aralkyl or aryl and

Y represents an electron-withdrawing leaving group, if appropriate in the presence of a diluent, if appropriate in the presence of an acid-binding agent and if appropriate in the presence of a phase-transfer catalyst.

4. Agents for combating pests, characterized in that they contain at least one substituted pyrazolin-5-one of the formula (I) according to Claims 1 and 3.

5. Use of substituted pyrazolin-5-ones of the formula (I) according to Claims 1 and 3 for combating pests.

6. Method of combating pests, characterized in that substituted pyrazolin-5-ones of the formula (I) according to Claims 1 and 3 are allowed to act on pests and/or their environment.

7. Process for the preparation of agents for combating pests, characterized in that substituted pyrazolin-5-ones of the formula (I) according to Claims 1 and 3 are mixed with extenders and/or surface-active agents.

**Revendications**

1. Pyrazoline-5-ones substituées de formule (I)

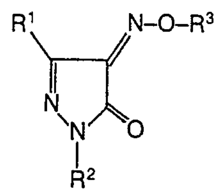

(I)

dans laquelle

$R^1$ représente de l'hydrogène ou un alcoyle à chaîne droite ou ramifiée, comportant 1 à 8 atomes de carbone

$R^2$ de l'hydrogène, un alcoyle, alcényle, alcinyle, cyanoalcoyle, hydroxyalcoyle, alcoxyalcoyle, hydroxycarbonylalcoyle, alcoxycarbonylalcoyle, aminocarbonylalcoyle, alcoylaminocarbonylalcoyle ou dialcoylaminocarbonylalcoyle, chaque fois à chaîne droite ou ramifiée et ayant chaque fois jusqu'à 8 atomes de carbone dans les diverses portions alcoyle, alcényle ou alcinyle, un oxyranylalcoyle ayant 1 à 4 atomes de carbone dans la portion alcoyle à chaîne droite ou ramifiée, un aralcoyle à chaîne droite ou ramifiée, éventuellement substitué une ou plusieurs fois, de manière identique ou différente, ayant 1 à 4 atomes de carbone dans la portion alcoyle et 6 à 10 atomes de carbone dans la portion aryle, ou un aryle éventuellement substitué une ou plusieurs fois, de manière identique ou différente et avec 6 à 10 atomes de carbone, en envisageant chaque fois comme substituants: halogène, cyano, nitro, hydroxy, alcoyle, alcoxy, dioxyalcoylène, alcoylcarbonyloxy ou alcoylthio chaque fois à chaîne droite ou ramifiée avec chaque fois jusqu'à 4 atomes de carbone, halogénoalcoyle, halogénoalcoxy ou halogénoalcoylthio ayant chaque fois jusqu'à 4 atomes de carbone et jusqu'à 9 atomes d'halogène identiques ou différents, ou un phényle, et

$R^3$ un alcoyle à chaîne droite ou ramifiée ayant 1 à 20 atomes de carbone, un alcényle, alcinyle, cyanoalcoyle, hydroxyalcoyle, alcoxyalcoyle, hydrocarbonylalcoyle ou alcoxycarbonylalcoyle, chaque fois à chaîne droite ou ramifiée avec chaque fois jusqu'à 8 atomes de carbone dans les diverses portions alcoyle, alcényle ou alcinyle, un cycloalcoyle ayant 3 à 7 atomes de carbone, cycloalcoylalcoyle ayant 3 à 6 atomes dans la portion cycloalcoyle et 1 ou 2 atomes de carbone dans la portion alcoyle, ou un arylalcoyle, aryloxyalcoyle ou arylthioalcoyle substitué chaque fois éventuellement une ou plusieurs fois de manière identique ou différente, tout en ayant 1 à 4 atomes de carbone dans les diverses portions alcoyle et 6 à 10 atomes de carbone dans les portions aryle, en envisageant comme substituants ceux qui sont cités dans $R^2$, $R^3$ toutefois ne signifiant un méthyle ou un éthyle que lorsque $R^1$ et/ou $R^2$ ne sont pas en même temps de l'hydrogène ou un méthyle, étant excepté en outre le composé 1-phényl-3-méthyl-4-benzyloximino-5-pyrazolone.

2. Pyrazoline-5-ones substituées selon la revendication 1, dans la formule (I)

$R^1$ étant de l'hydrogène, un méthyle, éthyle, n- ou i-propyle et n-, i-, s- ou t-butyle,

$R^2$ de l'hydrogène, un méthyle, éthyle, n- ou i-propyle, n-, i-, s- ou t-butyle, allyle, butényle, propargyle, cyanométhyle, cyanoéthyle, hydroxyméthyle, hydroxyéthyle, méthoxyméthyle, méthoxyéthyle, éthoxyméthyle, éthoxyéthyle, hydroxycarbonylméthyle, hydroxycarbonyléthyle, méthoxycarbonylméthyle, méthoxycarbonyléthyle, éthoxycarbonylméthyle, éthoxycarbonyléthyle, aminocarbonylméthyle, méthylaminocarbonylméthyle, éthylaminocarbonyléthyle, éthylaminocarbonylméthyle, diméthylaminocarbonylméthyle, diéthylaminocarbonylméthyle, aminocarbonyléthyle, oxiranylméthyle, oxiranyléthyle ou un phényle ou benzyle éventuellement substitué chacun une à trois fois, de manière identique ou différente, en envisageant comme substituants: fluor, chlore, brome, iode, cyano, nitro, hydroxy, méthyle, éthyle, n- ou i-propyle, n-, i-, s- ou t-butyle, méthoxy, éthoxy, dioxyméthylène, dioxyéthylène, méthylthio, éthylthio, acétoxy ou propyonyloxy, chlorométhyle, dichlorométhyle, trichlorométhyle, trifluorométhyle, dichlorofluorométhyle, chlorodifluorométhyle, trifluorométhoxy, trifluorométhylthio ou phényle et

$R^3$ un méthyle, éthyle, n- ou i-propyle, n- ou i-butyle, n- ou i-pentyle, n- ou i-hexyle, n- ou i-heptyle, n- ou i-octyle, n- ou i-nonyle, n- ou i-décyle, n- ou i-dodécyle, n- ou i-hexadécyle, allyle, butényle, propargyle, cyanométhyle, cyanoéthyle, hydroxyméthyle, hydroxyéthyle, méthoxyméthyle, méthoxyéthyle, éthoxyméthyle, éthoxyéthyle, hydroxycarbonylméthyle, hydroxycarbonyléthyle, méthoxycarbonylméthyle, méthoxycarbonyléthyle, éthoxycarbonylméthyle, éthoxycarbonyléthyle, cyclopropyle, cyclopentyle, cyclohexyle, cyclohexylméthyle, ou un radical éventuellement substitué une à trois fois, de manière identique ou différente, de formules

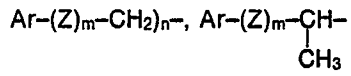

ou

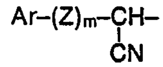

Ar représentant chaque fois un phényle ou naphtyle,

Z représentant chaque fois de l'oxygène ou du soufre,

m représentant 0 ou 1 et

n représentant 1, 2 ou 3,

en envisageant comme substituants ceux qui ont été cités pour $R^2$, avec la limitation que $R^3$ ne représente un méthyle ou éthyle que si $R^1$ et $R^2$ ne représentent pas simultanément de l'hydrogène ou un méthyle, faisant exception en outre le composé 1-phényl-3-méthyl-4-benzyloximino-5-pyrazolone.

3. Procédé de fabrication de pyrazoline-5-ones substituées de formule (I)

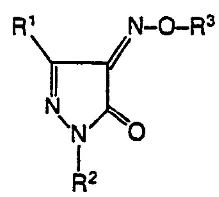

(I)

dans laquelle

$R^1$ représente de l'hydrogène ou un alcoyle à chaîne droite ou ramifiée ayant 1 à 8 atomes de carbone,

$R^2$ représente de l'hydrogène, un alcoyle, alcényle, alcinyle, cyanoalcoyle, hydroxyalcoyle, alcoxyalcoyle, hydroxycarbonylalcoyle, alcoxycarbonylalcoyle, aminocarbonylalcoyle, alcoylaminocarbonylalcoyle ou dialcoylaminocarbonylalcoyle, chacun à chaîne droite ou ramifiée et ayant chacun jusqu'à 8 atomes de carbone dans les diverses portions alcoyle, alcényle ou alcinyle, un oxiranylalcoyle ayant 1 à 4 atomes de carbone dans la portion alcoyle à chaîne droite ou ramifiée, un aralcoyle à chaîne droite ou ramifiée éventuellement substitué une ou plusieurs fois, de manière identique ou différente et avec 1 à 4 atomes de carbone dans la portion alcoyle et 6 à 10 atomes de carbone dans la portion aryle ou un aryle substitué éventuellement une ou plusieurs fois de manière identique ou différente et comportant 6 à 10 atomes de carbone, en envisageant chaque fois comme substituants: halogène, cyano, nitro, hydroxy, un alcoyle, alcoxy, dioxyalcoylène, alcoylcarbonyloxy ou alcoylthio, chacun à chaîne droite ou ramifiée et comportant chaque fois jusqu'à 4 atomes de carbone, un halogénoalcoyle, halogénoalcoxy ou halogénoalcoylthio, à chaîne droite ou ramifiée, chaque fois avec jusqu'à 4 atomes de carbone et jusqu'à 9 atomes d'halogène identiques ou différents, ou un phényle, et

$R^3$ un alcoyle à chaîne droite ou ramifiée ayant 1 à 20 atomes de carbone, un alcényle, alcinyle, cyanoalcoyle, hydroxyalcoyle, alcoxyalcoyle, hydroxycarbonylalcoyle ou alcoxycarbonylalcoyle chacun à chaîne droite ou ramifiée et comportant chaque fois jusqu'à 8 atomes de carbone dans les diverses portions alcoyle, alcényle ou alcinyle, un cycloalcoyle ayant 3 à 7 atomes de carbone, un cycloalcoylalcoyle ayant 3 à 6 atomes de carbone dans la portion cycloalcoyle et 1 ou 2 atomes de carbone dans la portion alcoyle, ou bien un arylalcoyle, aryloxyalcoyle ou arylthioalcoyle éventuellement substitué chacun une ou plusieurs fois, de manière identique ou différente et avec 1 à 4 atomes de carbone dans les diverses portions alcoyle et 6 à 10 atomes de carbone dans les portions aryle, en envisageant comme substituants ceux qui ont été cités pour $R^2$, $R^3$ toutefois ne représentant un méthyle ou éthyle que si $R^1$ et/ou $R^2$ ne représentent pas simultanément de l'hydrogène ou un méthyle, faisant exception aussi le composé 1-phényl-3-méthyl-4-benzyloximino-5-pyrazolone, caractérisé en ce que

(a) on fait réagir des 4-oximino-pyrazoline-5-ones de formue (II)

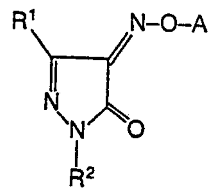

(II)

dans laquelle

$R^1$ et $R^2$ ont la signification indiquée plus haut et

A représente de l'hydrogène ou un cation de métal alcalin,

avec des agents d'alcoylation de formule (III),

$$R^3\text{–}X \qquad \text{(III)}$$

dans laquelle

$R^3$ a la signification indiquée plus haut et

X représente un groupe de départ électrophile, éventuellement en présence d'un diluant, éventuellement en présence d'un agent fixateur d'acide et éventuellement en présence d'un catalyseur de transfert de phase, ou

(b) en ce qu'on fait réagir les 4-alcoximinopyrazoline-5-ones de formule (Ia) obtenables par le procédé (a)

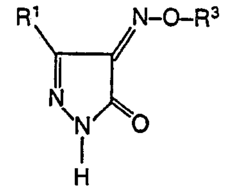

(Ia)

dans laquelle

$R^1$ et $R^3$ ont la signification indiquée plus haut, avec des agents d'alcoylation de formule (IV)

$$R^{2'}\text{–}Y \qquad \text{(IV)}$$

dans laquelle

$R^{2'}$ représente un alcoyle, alcényle, alcinyle, cyanoalcoyle, hydroxyalcoyle, alcoxyalcoyle, hydroxycarbonylalcoyle, alcoxycarbonylalcoyle ou bien un aralcoyle ou aryle éventuellement substitué chacun et

Y représente un groupe de départ électrophile, éventuellement en présence d'un diluant, éventuellement en présence d'un agent fixateur d'acide et éventuellement en présence d'un catalyseur de transfert de phase.

4. Agent antiparasitaire, caractérisé par une teneur en au moins une pyrazoline-5-one substituée de formule (I) selon les revendications 1 et 3.

5. Utilisation de pyrazoline-5-ones substituées de formule (I) selon les revendications 1 et 3 pour combattre les parasites.

6. Procédé pour combattre les parasites, caractérisé en ce qu'on fait agir des pyrazoline-5-ones substituées de formule (I) selon les revendications 1 et 3 sur les parasites et/ou sur leur espace vital.

7. Procédé de fabrication d'agents antiparasitaires, caractérisé en ce qu'on mélange des pyrazoline-5-ones substituées de formule (I) selon les revendications 1 et 3 avec des diluants et/ou des agents tensioactifs.